# EUROPEAN PATENT SPECIFICATION

(11) **EP 3 752 112 B1**
(45) Date of publication and mention of the grant of the patent: **15.10.2025**
(21) Application number: 19706264.9
(22) Date of filing: 15.02.2019
(51) Int. Cl.: A61F 13/15, A61F 13/47, A61F 13/472

(54) **AN ABSORBENT MEMBER FOR A BODY CLEFT ADAPTED PRODUCT AND PRODUCT WITH SUCH ABSORBENT MEMBER**
ABSORBIERENDES ELEMENT FÜR EIN AN KÖRPERSPALTEN ANGEPASSTES PRODUKT UND PRODUKT MIT EINEM SOLCHEN ABSORBIERENDEN ELEMENT
ÉLÉMENT ABSORBANT POUR UN PRODUIT ADAPTÉ À UNE FENTE CORPORELLE ET PRODUIT COMPORTANT UN TEL ÉLÉMENT ABSORBANT

(30) Priority: 15.02.2018 GB 201802453
(43) Date of publication of application: 23.12.2020
(73) Proprietor: Blevins, John, 65719 Hofheim (DE)
(72) Inventor: Blevins, John, 65719 Hofheim (DE)
(74) Representative: Plischke, Manfred
(86) International application number: PCT/EP2019/053752
(87) International publication number: WO 2019/158672

(56) References cited:
- EP-A1- 1 407 739
- EP-A1- 1 543 806
- US-A1- 2005 277 903

## Description

### FIELD OF THE INVENTION

This invention relates to absorbent members for absorbent devices or articles as well as to such absorbent devices or articles, more particularly in the context of improved absorbent devices that are worn in body creases or clefts, such as interlabially by female wearers for catamenial purposes, incontinence protection, or both, and in the anal crease for light faecal incontinence or in the context of haemorrhoids, or underarm sweat-pads.

### BACKGROUND OF THE INVENTION

Absorbent products, articles or devices that are adapted for being fitted into body clefts, creases or similar parts of human bodies as well as absorbent member for such products, articles or devices are well known in the art.

A first category relates to articles positioned in anal region as anal pad or haemorrhage treatment pad for absorbing anal exudates or sweat, for minimizing the consequences of flatulence, or for applying substances like medicine in case of haemorrhages, see e.g. US4182335A of 1977, US6716229B2 (1997), US8062277B2. In US8353884B2 an anal patch for handling e.g. low level faecal incontinence and soiling in the anal area, is described, which has a pad with an absorbent member with a contact surface perpendicular to two flat surfaces, and adhesive backing layer with an aperture aligned with contact surface. In US8062277B2 a device is described for e.g. flatulence or leakage absorbency and the positioning of a non-adhesive intergluteal absorbent pad close to wearer's anal orifice such that the pad is retained frictionally between buttocks. WO2010072216A1 relates to an insert for items of clothing made of a material which can absorb sweat comprising cellulose and breathable material, and which may be worn as axillary pad.

With respect to feminine protection devices, there are well known sanitary napkins for external wear about the pudendal region and tampons for internal wear within the vaginal cavity for interruption of menstrual flow therefrom. Also hybrid devices which attempt to merge the structural features of sanitary napkins and tampons into a single device have been proposed, see e.g. US2092346 from 1937, or US3905372 from 1975.

Another approach for less intrusive hybrid devices became known as interlabial pads. Such pads have a portion which at least partially resides within the wearer's vestibule and a portion which at least partially resides external of the wearer's vestibule, see e.g. US2662527 from 1953 or US4631062 from 1986.

Further examples of such absorbent members and devices are described in U.S. Pat. No. 2,917,049 issued to Delaney on Dec. 15, 1959, U.S. Pat. No. 3,420,235 issued to Harmon on Jan. 7, 1969, U.S. Pat. No. 4,595,392 issued to Johnson, et al. on Jun. 17, 1986, and U.S. Pat. Nos. 5,074,855 and 5,336,208 issued to Rosenbluth, et al. on Dec. 24, 1991 and Aug. 9, 1994 respectively, and U.S. Pat. No. 5,484,429 issued to Vukos, et al. on Jan. 16, 1996.

In EP1543806, an inter-labia pad is described comprising an absorbing including a labia-following layer and a bodily liquid-storing layer. The labia-following layer, together with the cover material, deforms in accordance with the shape of the wearer's labia, and the bodily liquid storing-layer is in contact with the labia-following layer so that bodily liquid can move from the labia-following layer to the liquid-storing layer.

EP1407739 describes an interlabial pad with a finger insertion opening, which is formed along the longitudinal direction of a back side sheet between a mini sheet piece and the side opposite to body on the back side sheet to allow easy application of the pad.

One of few interlabial devices products that became commercially available was "FRESH 'N FIT PADETTE" (also known as "INSYNC" or "INSYNC MINIFORM") interlabial pad, which was marketed by Athena Medical Corp. of Portland, Oregon, US, and which was described in US3983873 and US4175561. Similarly a product "Sofy Syncro Fit" has been offered by UNICHARM Corp (JP) as adding absorbency for menses in combination with an absorbent pad.

However, such products still have no broad commercial distribution, which appears to be the result of several factors, including complex manufacturing processes and inefficient material utilization leading to high manufacturing costs.

Further and more recently with much higher relevance, such products, as being typically disposed of via toilets, should preferably satisfy more and more stringent requirements for flushability to not disturb the sewage systems as well as providing biodegradability in case that the sewage treatment system does not allow for capturing flushed pads.

Therefore, a need exists for an improved absorbent article which will address these problems and reduce the incidence of body and panty soiling when used. Such a device should be easy to insert and comfortable during wear. A need exists for an interlabial device which also covers the walls of the wearer's labia throughout a range of body motions and reliably covers the vaginal introitus and preferably also the urethra during such motions. Preferably, the article is adapted to receive the bodily exudates on the side portions of the product rather than primarily over the folding ridge, which can be well assesses by applying the Folded Capacity Test or its respective subtests.

### SUMMARY

Thus, in a first aspect, the present invention is an absorbent article for being worn in a human body cleft, preferably between female labia, between buttocks or gluteal clefts, or in the armpit. The article comprises an absorbent member, such that one or more of the following conditions are satisfied upon execution of the "Folded Capacity Tests" as described herein below:
- the article exhibits
   ∘ a high pressure drip off capacity under 3.92 kPa (also expressed as 40 g/cm² load) of at least 2 ml;
   ∘ low pressure drip off capacity under 0.981 kPa (also expressed as 10 g/cm²) of at least 4 ml;
   ∘ high pressure edge wetting of no more than 0.1 g at a test fluid load of 80 % of high pressure drip off capacity under 3.92 kPa;
   ∘ low pressure edge wetting of no more than 0.1 g at a test fluid load of 80 % of low pressure drip off capacity under 0.981 kPa
- the absorbent member exhibits
   ∘ a high pressure drip off capacity factor under 3.92 kPa (also expressed as 40 g/cm² load) of at least 2 ml / g of core material;
   ∘ a low pressure core drip off capacity factor under 0.981 kPa (also expressed as 10 g/cm² load) of at least 4 ml per gram of core material.

The article preferably consists of compounds that are essentially fully biodegradable. The article may exhibit an in-use configuration, wherein the article comprises a fold line adapted to be positioned parallel to a sagittal plane of a wearer, thereby defining the longitudinal direction of the article. A first and a second side portion may extend away from the fold-line in the width direction of the article, preferably at an angle of less than 45° between them. The side portions may optionally exhibit an essentially identical shape, symmetrical to the longitudinal fold line. Each of the side portions may comprise a first surface adapted to be in direct contact with the skin of a wearer, to receive bodily exudates emitted from a wearer, and being formed by a topsheet material, and a second surface opposite of the first surface, the second surface being spaced apart from the first surface by the thickness of the side portion, and the second surface being formed from a backsheet raw material exhibiting a backsheet raw material strength, and an absorbent core positioned between the first and the second surface. Preferably, the backsheet and the topsheet materials of the first and second side portions are of the same material type, more preferably unitary, and the article is adapted to maintain an in-use configuration throughout the anticipated usage periods.

In a pre-use configuration, the article may be pre-folded along the fold line, partially folded along the fold-line, or unfolded, preferably comprising a folding aid for easing transformation into the in-use configuration by a user.

An article according to the present invention comprises
i) a topsheet based on natural materials,;
ii) an absorbent core comprising fibers and superabsorbent material;
iii) a backsheet based on natural or degradable materials.

The topsheet, absorbent core, or backsheet comprise compounds selected from the group consisting of
- cellulose fibers or modified cellulose materials;
- starch, starch based or starch derived compounds;
- polylactic acid or polylactates;
- polyhydroxy-butyrate, - alkanoate, or - valerate;
- hydroxymethylfurfural;
- polycaprolactone;
- polyamide;
- polyurethane.

Further an article according to the present invention may comprise one or more of the elements selected from the group consisting of
iv) a removal means, preferably strings, stripes or bands;
v) an application aids, preferably finger pouch;
vi) an attachment means for temporarily connecting a surface of the article to other surfaces, preferably of garments or skin of the wearer;
vii) an emollient / lotion applied to the first surface;
viii) edge barrier webs;
ix) a barrier compound applied to the edges, preferably a wax;
ix) an absorbent core with a rounded shape at least at its longitudinal ends, preferably comprising two sub-layers;
x) being part of a series of articles, preferably wrapped articles, wound up in a roll, the roll preferably exhibiting the width of conventional toilet paper rolls, the series of articles being arrange in a single or multi lane arrangement, preferably a staggered multi-lane arrangement.

An article according to the present invention further
exhibits an overall length of
   more than 50 mm, preferably more than 70mm,
   but less than 150mm, preferably less than 120 mm, more preferably less than 110 mm,
and an overall width of
   more than 30 mm, preferably more than 40 mm,
   but less than 150 mm, preferably less than 130 mm;

An article according to the present invention is essentially free of adhesives, preferably comprising thermofusible bonding only.

In a particular execution, an absorbent article according to the present invention may comprise an absorbent core comprising
a first piece of a first absorbent web material, and
a second piece of a second absorbent web material,
each of the first and the second pieces exhibiting
   a first and a second end edge
   opposite along the longitudinal direction of the absorbent core,
   connected by longitudinally extending side edges,
wherein the first and the second piece being positioned z-directionally in a partly overlapping arrangement, and longitudinally in an offset arrangement.

### BRIEF DESCRIPTION OF THE FIGURES

Fig. 1A to C depict conventional articles suitable to be worn in body clefts.
Fig. 2 A to F explain certain embodiments that may be employed in articles according to the present invention.
Fig. 3 A to C depict a pre-use configuration for an article according to the present invention.
Fig. 4 shows specific elements that may be employed with an article according to the present invention.
Fig. 5 depicts a particular execution of an article according to the present invention.
Fig. 6A to C depict particular executions according to the present invention.
Fig. 7 A to D depict a particular execution of an absorbent core according to the present invention.
Fig. 8 A and B depict further elements suitable for articles according to the present invention.
Fig. 9 A and B depict executions of absorbent cores according to the present invention.
Fig. 10 A and B depict a particular execution of an article according to the present invention.
Fig. 11 A to E show details for test stand for evaluating absorbent cores or articles according to the present invention.
Fig. 12 depicts a further particular execution of an article according to the present invention.

Same numerals refer to same or equivalent elements or features.

### DETAILED DESCRIPTION

The present invention is an absorbent device that is intended to be worn in a body cleft, in particular interlabially by female users or in the intergluteal cleft of the buttocks in case of light fecal incontinence or bleeding haemorrhoids or an underarm sweat pad.

The term "device" is used herein essentially interchangeably with "article" or "product" and refers to items that are essentially ready to be used by a user. Absorbent devices comprise an absorbent member, interchangeably also referred to as absorbent core, which comprises absorbent materials and provides absorbency to the device. Typically, an absorbent device comprises an absorbent member or core that is positioned between a topsheet, that is intended to be positioned towards a wearer and contacting the skin of a wearer, and a backsheet, forming the opposite surface of the device or article. Typically, the top- and backsheet are clearly distinct from the absorbent member or core, though it is also within the scope of the present invention that the topsheet and backsheet form integral surface layers of a composite with the absorbent member.

The present invention relates to an absorbent article as well as to an absorbent core for such absorbent article for absorbing bodily exudates such as urine, faeces, menses, sweat, and the like in particular in body clefts. Thus articles according to the present invention or comprising an absorbent core according to the present invention may be feminine hygiene pads, so called interlabial pads, or sweat pads, as may also be referred to as axillary pads or pads for armpits, or haemorrhoidal pads. Typically all such articles are intended for a single use to be disposed thereafter in an environmentally compatible manner. Henceforth, for particular applications it might be desirable that the article and its ingredients are flushable, disintegratable, compostable, or biodegradable.

The product according to the present invention is adapted to have three configurations, a pre-, an in-, and a post-use configuration.

In the "pre-use configuration" the product is as manufactured by a producer and packed into a packed form. The package format may be single wrap, box, roll and the article may be fully or partially folded or essentially flat. Both the article and the package are adapted to withstand the usual handling and transporting from the manufacturer to the user without substantially changing the appearance or properties of the article.

Preferably, the present product allows easy manufacturing with minimal cost and resources, such as by minimizing trim removal of material to the utmost extent.

The "in-use configuration" refers to a product as taken from the package and applied to the body by a user. To this end, the user may take the article from the box, remove the single wrap, or take it from a roll. The user may fold the product or retain a folded shape of the "pre-use configuration" such that it can be suitably positioned relative to the body.

The usage period may be up to two hours, often will be about four hours or may even be over eight hours, e.g. during overnight usage, depending on
(i) the intended use, e.g. during heavy or low flow menstruation periods, or to capture urine at drip incontinence or normal incontinence, or to be used as single protection against leakage or in combination with a further absorbent article, and
(ii) on the design capacity as may be manufactured into and indicated on the package or article by the manufacturer.

During use, the article is adapted to receive and retain bodily exudates, but to not release any harmful substances that may reach the body.

The "post-use configuration" refers to the article as removed from the body for disposal. In the post-use configuration the article is preferably adapted for both solid waste disposal and sewage waste disposal.

For the latter, the article shall be flushable, i.e. not clogging the sewage system, and preferably is disintegratable, i.e. losing its pre- and in-use configuration shape and structure. Preferably, the materials of the article are degradable, such as in sewage systems, but also in industrial composting, bio-gasification systems or the like or even under uncontrolled disposal in the environment.

It should be noted that in the post-use configuration, the article may not exhibit a particular article shape, but may have been separated, such as by disintegration or dissolving, into several or many article remains, that may or may not be discernible in a sewage or waste treatment system.

The product provides benefits at all stages of its life cycle to the manufacturer, to the user and to the environment by exhibiting a novel combination of properties for each of the configurations, as may be achieved by particular selection and combination of conventional approaches for material selection, combining and bonding mechanisms, and design elements.

### Properties to be satisfied

It is a particular feature of the present invention that various seemingly contradicting constraints for various properties are put together, in particular relating to the behavior upon contacting liquids.

As to a first property, the article is adapted to absorb bodily liquids in its in-use configuration. To this end, it comprises absorbent materials such as in general well known to a person skilled in the art of hygiene products, such as absorbent fibers, such as natural fibers (including chemically modified natural material based fibers) or synthetic fibers, or superabsorbent materials, in particulate or fibrous form, being purely synthetic material based, such as the broadly used poly-acrylate based materials, or be these manufactured with renewable resource materials such as starch-grafted materials.

The absorbency of an article is typically determined by considering the functionality of the article with regard to
- receiving the liquid (acquisition),
- distributing the liquid (distribution) and
- ultimately storing the liquid (absorbent capacity)
- with little or no liquid release during use (rewet).

The overall absorbency of the article is adapted to its intended use, and may range from few milliliters to up to 100 ml per article in the in-use configuration.

Absorbency may be determined by various methods. A simple approach is to approximate it by "theoretical capacity", that will be based on the sum of the absorbency of the materials for 0.9% saline solution in the "centrifuge capacity test" for absorbent materials. Absorbency may also be determined for the article in its in-use configuration. As for the present application the in-use configuration may be a folded one, and the "in-use absorbency" may be determined by submitting the article to the "Folded Capacity Test" as described herein below.

Further, the article may be described by its "design capacity" and may be derived from expected or intended loading for the particular application. For example, the design capacity may be for various intended uses for protection
- in case of drip incontinence for a day-time use, i.e. over 2 to 4 hours, or for bleeding haemorrhoids the design capacity may be as low as 2 ml;
- for overnight heavy flow menstrual condition 10 ml, or
- for light urine incontinence up to 100 ml.

Based on such design capacity, the theoretical and in-use capacity allow for a safety margin of 10%, 15% or even 50% of the design capacity.

In case that no design capacity is given, but a theoretical or in-use capacity can be determined, the design capacity is set to be 80% thereof.

With regard to receiving and acquiring bodily liquids, the articles according to the present invention typically pose a less stringent requirement as for example urine receiving diapers do, as the surface of the article being positioned in the cleft between the labia or of the buttocks the surface is generally in good contact with the skin of the wearer.

It is a particular feature of articles according to the present invention that they are applied an a folded configuration, with the folding ridge region comprising the fold line oriented towards the body cleft, and the side portions extending away from there. Further the liquid load may very well be transferred along the skin of the wearer towards the side portions of the article, rather than the folding ridge region. Also the pressure as applied by the wearer may vary broadly, and may range from 3.92 kPa (also expressed as 40 g/cm² load), under which typically absorbent products are evaluated, down to 0.981 kPa (also expressed as 10 g/cm²). Henceforth an article should suitably exhibit a "Folded Article Drip Off Capacity" at either pressure of typically more than 0.5 ml, preferably more than 1 ml, often more than 3 ml, and for certain applications also well over 10 or even 20 ml. However, as the physical space in the body cleft is often limited and the absorbed liquid corresponds to a certain volume, the basis capacity, as expressed in milliliter per unit area, should not be excessively high, as this will then also determine the thickness of a loaded article, that should be less than 20 mm, preferably less than 10 mm, and is often less than 5 mm.

Further, the articles should exhibit a good edge wetting performance, as tested by measuring the wetting of filter paper positioned at the lower edge of the article at a predetermined loading.

With regard to surface wetness, as may be determined by an adaptation of the well-established post acquisition rewet test in a the "Post Drip Off Capacity Rewet Test", for certain applications articles according to the present invention do not need to exhibit maximized dryness performance, and the sensitive skin which the articles are contacting may even react negatively if overly dried out. As will be discussed herein below, this may be achieved by permanently hydrophilic topsheet material, such as made from or comprising natural materials like cotton or natural material based materials such as viscose.

A second class of properties for which the article shall satisfy particular requirements is the mechanical properties. Thus, the article is adapted to withstand in the in-use configuration the mechanical stress as may be exerted thereto during use, as well as staying in place during use.

This refers to the condition of the article being in an unloaded condition, i.e. in the "pre-use" configuration such as the last manufacturing or packaging and transportation steps, but also in the "in-use" configuration before the first wetting occurs, as well as in a loaded condition, at least up to its design capacity, preferably also beyond.

This can be determined by submitting the article to a tensile test, either in a flat or in a folded configuration, and also when being loaded with fluids or even aged after loading.

In addition or alternatively, the friction between contacting backsheet materials - both under dry and wet conditions should neither be too low, which might create a slippery feel and or give raise to difficulties for applying the article or for staying in place, nor be too high, which might result in undesired dislocation during use.

A third class of properties relates to the users health, in particular that the article does not irritate the skin of the wearer, in particular when used as an interlabial pad. To this end, the article has a soft surface, both under dry (i.e. immediately after application) and under wet conditions (upon and after wetting). Further the article should not release any undesired substances to the wearer. Henceforth the article should exhibit a low fuzz value, such as may be established according to the test methods as described herein below. Also the article should have low levels of extractable matter if submitted to a non-stirred 0.9% saline or distilled water extraction over 2, 4, and 12 hours at room temperature or even more at 37°C.

In an optional set up, the article may comprise substances for improving the conditions of the skin or easing the application, such as lotions, emollients and the like. Whilst articles according to the present invention may comprise further ingredients such as odor enhancers or even medicine delivering formulations, these should be applied so as to not compromise the health of the wearer.

Yet a fourth class of properties is related to flushability, i.e. that the article is adapted to be flushable in various types of toilets. Whilst there exist many types of toilet designs and sewage handling and treatment systems, it may be well sufficient if the articles according to the present invention pass through such sewage handling system without mayor degradation or disintegration because of their size for being removed at a sewage treatment plant. Optionally, the articles disintegrate so as to ease handling in such systems, however, in this case they should also exhibit degradability properties (see test section herein below) so as to not create undesired "micro plastics".

The compatibility with sewage treatment systems and the disintegration, if present, may preferably be assessed by flushability tests and criteria, in particular as developed by INDA /EDANA for articles like wipes and articles according to the present invention. If needed, the testing protocol might be adjusted, e.g. to allow for the size of the absorbent articles that might be smaller than the minimum size required in the testing protocol.

To pass the test, an article has to disintegrate very quickly, at least to article portions that will then allow good flushing. As will be discussed in more detail, the flushability may be established by the combination of material selection and configuration.

Whilst the disposal route through the sewage water treatment system, may be a preferred one, articles according to the present invention should not release any dangerous or potentially hazardous material or compounds to the environment, if they are disposed in a solid waste treatment system, as may be incineration, composting, landfilling, or even uncontrolled disposal as still being practiced in certain regions.

A sixth class of properties relates to degradability, more preferably biodegradability. Once having entered the sewage system, or the solid waste treatment system, or even under uncontrolled disposal conditions, it is often highly preferred, that the article or its remains in the post-use configuration are to a large extent or even fully degradable, i.e. ultimately converted into carbon dioxide and water. **In** particular it is highly undesirable that non-degradable residues reach the ecological systems such as in the form of non-degradable "micro-plastics". Most preferably, such a biodegradability of the article is established by selection of the raw materials for the making of components of the article according to the present invention, though it is conceivable that this may be achieved by the addition of decomposition aids.

### Definitions

All percentages [%] are given as "% by weight", unless expressly mentioned differently.

The "configuration of an article" refers to the physical structure of the article as defined by the design of the article and established by combining various materials forming the shape of the article. For a made article, the configuration further refers to the set-up of an article, e.g. a given article may be in a flat or in a folded state, or a feature or an element of the article may be inwardly or outwardly folded.

One article may take various configurations and for each thereof different properties may be relevant. Also, a given property may be different in different configurations.

An article in a flat configuration exhibits a length (x-) and a width (y-) direction, and a thickness (z-) direction perpendicular thereto. Unless expressly stated, the length direction is coinciding with an anterior - posterior orientation on a wearer, i.e. parallel to the sagittal plane of a wearer, which includes the interlabial space, the anal cleft and axillaries or armpits. Accordingly, the width direction is along the medial-lateral orientation on a wearer, i.e. perpendicular to a sagittal plane of a wearer.

Typically though not necessarily, the article exhibits a shape that is symmetrical to its longitudinal centerline. As the thickness of a flat article is typically significantly smaller than the other two dimensions, an article can be viewed in a top view as a two dimensional item, and the article or segments or portions thereof is/are delimited by the edges thereof. Thus, an article has a front and a rear edge and side edges connecting the two.

The transition from one edge to another neighboring one, e.g. from the front edge to a side edge, is typically clearly visible as a corner or angle when the edges are formed by at least one straight line(s) or discontinuous curves (e.g. circles differing in radius and not having a joint tangent) in the vicinity of the transition corner.

However when the edges transition gradually, such as for example in the case of an egg-shaped article, the transition points are defined as being an intersection of the perimeter of the article with two lines parallel to the longitudinal centerline but laterally offset by 40% of the maximum width of the article.

Unless referred to expressly, the term "edges" generally refers to the outer edges of a structure, whilst "inner edges", such as at the perimeter around the hole may exist in a structure.

When referring to a "flat configuration" of the article, this does, however comprise structures made of several z-directionally distinct layers of same or different dimension, for example when a backsheet and a topsheet envelop an absorbent core thereby exhibiting a larger size than the core such that a margin extends around the edges of the core towards the edges of the topsheet or backsheet, respectively. Similarly, a core may comprise two or more layers in a z-directional arrangement, that together form the overall core structure with the respective core edges. Thus, the term "flat" does not necessarily require a constant thickness.

It also should be noted that the topsheet and/or backsheet materials may extend to the respective other surface of the article. For example, the backsheet may be folded around the edges so as to create a perimeter sealing around a core.

An essentially flat structure, such as a flat article may be transformed from a flat configuration into a folded configuration along a fold line, from which side portions extend laterally.

The skilled reader will readily realize that then the "z-direction" will be dependent where - relative to the fold line - the article is considered, and that then "z-direction" corresponds to the caliper of the article or material.

Thus, if an article comprises for example three layers of material, e.g. in a z-directional cross-sectional view a topsheet, a backsheet and a core layer there between, the folded configuration may show in such a view a topsheet, a core layer, two backsheet layers, a core layer, and a topsheet.

It should be noted, that not necessarily all layers need to be present in the fold line, such that e.g. for easier folding, the core layer is not present in the vicinity of the fold line. Similarly, such a folded structure can also be made by placing mirror-symmetric structures onto each other, e.g. two topsheet-core-backsheet structures with the backsheets oriented towards each other, and connecting the two structures, such as along a bond line.

The side portions may extend laterally away from the fold line by being essentially flat and extending away along a straight line, thereby forming a sharp folding angle at the fold line, which may be essentially zero upon full overfolding. When being positioned on the body, the angle may be larger, e.g. when looking at a positioning in an armpit when arms are raised, but typically the folding angle will be less than 45°.

The term fold line includes an upper ridge line, e.g. in case the article is more shaped in an (inverted) U-shape, such as may occur when the side portions exhibit a certain thickness in the fold line preventing a sharp kink. In this case, the folding angle will be determined by connecting the upper ridge line with the laterally outermost point of the side portions. Within the present context, the term "topsheet" is used for a material layer or sublayer, that is intended to be oriented towards the skin of a wearer, and the term "backsheet" for a material positioned z-directionally opposite thereof. In particular in a folded in-use configuration, the cross-sectional view of an article may then take the form of a U or V or even essentially two parallel side portions connected at the folding or bond line. The topsheet, i.e. the outer surfaces of the folded article, may then be positioned towards the corresponding side portions of the body cleft, such as the labia or buttocks, whilst the opposite backsheet portions of the side portions are positioned towards each other, and may actually be in direct contact with each other.

### Material compounds

In order to arrive at articles according to the present invention, appropriate materials for the various components of the article should be selected, whereby various compounds allow balancing of key component requirements with regard to properties like solubility in aqueous medium, hydrophilicity or wettability, degradability, especially biodegradability, i.e. decomposition by enzymes or microbes, and related kinetics, thermoplastic properties like melt temperature, glass transition temperature, etc., mechanical properties (strength, elongation at break, brittleness. Also, the origin of the material may be relevant, such as coming from petroleum, natural - agricultural, or natural - microbial/ biotechnological sources.

Without intending particular restriction on the selection of suitable compounds, the following describes such materials as may be used alone or in combination.

A first category of such compounds is based on cellulose, such as cellulose fibers as such, or modified cellulose materials, such a Viscose ^{®} or Rayon ^{®} or cellulose esters, in particular when made into non-woven material or films.

Compounds may comprise starch, starch base, or starch derived materials, such as available from Novamont s.p.a, Italy, Biotec GmbH, Germany, Cerestech, Canada, Zhejiang Tianhe, China, Grace biotech, Taiwan, Japan corn starch, Japan, RKW SE, Germany.

Other compounds can comprise polylactic acid or polylactates, such as available from NatureWorks LLC, NE, USA, Tora Industries, JP, Teijin Ltd, JP, Zheijiang Hisun Biomaterials, CN, Nativia ^{®} of Taghleef Industries, Dubai, UAE, or RKW SE, Germany.

Further materials may be based on or comprise natural or degradable materials such as polyhydroxy butyrate (PHB), alkanoates (PHA), or valerates (PHBV), or Hydroxymethylfurfural, as also available from RKW SE, Germany, among others. Also, polycaprolactanes or certain polyamides or polyurethanes, may be suitably employed.

Other compounds that may be applied in the context of the present invention may be adhesives that exhibit designed solubility properties, such that they remain intact for a certain period or moisture exposure, but that start to lose their adhesive properties after a certain time after first wetting, or when an excess of water is provide, or another disintegration trigger is applied.

### Suitable components

The term "components" refers to compounds in a form that allow forming the absorbent article.

Within the present context, the term "web" or "web material" refers to an essentially endless material in one direction, i.e. its longitudinal extension, or the length, or the x-direction in Cartesian coordinates relative to the web material. Included in this term is an essentially unlimited sequence of pieces cut or otherwise separated from an essentially endless material. Typically the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. When the width of a web is reduced, it may form a band or even strings which are presently included under such a term.

Without intending any limitation, such web materials may be fibrous materials, such as cellulosic fiber materials, tissues, woven or non-woven materials, or other porous materials like absorbent foams, such as may be made as "high internal phase emulsion foams". Typically, though not necessarily, web materials are supplied in roll form, or on spools, or in a folded state in boxes. The individual deliveries may then be spliced together to form the essentially endless web. A web material may be composed of several (sub-)web materials, such as multilayer non-woven, coated tissues, non-woven / film laminates. Web materials may comprise other materials, such as added binding material, particles, in particular superabsorbent particles, hydrophilizing agents and the like.

If a web comprises fibers, these are typically bonded to each other or to other components of a web, such as other webs, such as films or fibrous webs exhibiting sufficient strength. There are many suitable bonding mechanisms available, such as thermal or melt fusion bonding, including ultrasonic bonding, or adhesive application.

Within the present context, webs are typically pre-fabricated, and may be stored and/or transported before being processed further, though it also within the scope that such webs may be formed in a separate process-step upstream of the presently considered web handling process. A web needs to sustain normal processing stresses, such as occur during handling, storage or transport and transfer, and thus pre-manufactured webs are typically pre-bonded.

Within the present context, the term unitary or integral refers to a component that comprises several sub-components, such as multiple layers which are formed in a single process or that are combined after made individually and combined into and delivered as a single materials to the process for manufacturing an article according to the present invention

Topsheet materials which are suitable for the present invention should satisfy the general requirements of being compliant, soft-feeling and non-irritating to the wearer's skin. Further, a topsheet is liquid permeable, permitting liquids to readily penetrate through its thickness. The topsheet has a body-facing side or face and a garment-facing side or face.

For certain applications, it may be desirable that a topsheet exhibits a balanced hydrophilicity so as to not create an overly dry feeling to the skin of a wearer. For certain applications, it may be desirable that a topsheet comprises or even essentially consists of natural compounds.

When a topsheet comprises or essentially consists of degradable compounds, it is desirable that the manufacturing of the topsheet does not impede this property.

A topsheet may comprise or essentially consist of a nonwoven web. A nonwoven web or fabric is a manufactured web of directionally or randomly oriented fibers, consolidated and bonded together by one or more patterns of bonds and bond impressions created through localized compression and/or application of heat or ultrasonic energy, or a combination thereof. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltspinning, meltblowing, spunlaying, solvent spinning, electrospinning, and carding. As used herein, "spunlaying" refers to fibers made by spunbond technology without having undergone further processing, such as bonding. The basis weight of nonwoven fabrics is usually expressed in grams per square meter (g/m² or gsm). A nonwoven web may be consolidated and bonded by hydroentanglement and/or needle punching, in addition or alternatively to being consolidated and bonded by bonds obtained by heat and/or compression (including ultrasonic bonding). A nonwoven web may be pattern bonded. Typically, the bond pattern is imparted by use of heat and/or pressure, and/or by ultrasonic bonding. Due to the use of heat, pressure and/or ultrasonic energy, the multicomponent fibers in the bond areas are pressed tightly together, which results in plastic deformation of the fibers. Especially if bonding is achieved by heat, or by heat and compression, the fibers in the bonded areas are molten, completely or partially, such that in the bonded areas the individual fibers are fused together (coalescence) to form a film-like structure.

A topsheet may comprise or essentially consist of an apertured film, such as being manufactured from a liquid impervious, preferably thermoplastic material. One suitable material is a low density polyethylene film having a thickness of from 0.001 to 0. 002 inches (0.0025to 0.0051 cm.). The thermoplastic material for use in the manufacture of the apertured plastic film is selected from a group of compounds as described in the above. The general melt indices for suitable compounds may range preferably from about 2 to about 100, with the more preferred range being from about 4 to about 25.

Preferably, such an apertured film may be a three-dimensional structure which has a plurality of tapered capillaries, each of which has a base opening and an apex opening, the latter being oriented towards an underlying layer, such as the absorbent core.

In a further execution, the topsheet may be a particular materials layer unitary with the absorbent core.

Backsheet materials suitable for the present invention need to be flexible so as to be compliant and will readily conform to the general shape and contours of the human body.

For certain applications it may be preferred, that the backsheet material prevents the exudates absorbed and contained in the absorbent core from wetting articles which contact the absorbent article. For certain applications, it may be preferred that the backsheet be "breathable", i.e. permitting vapours, especially water vapour to escape from the absorbent article, whilst preventing liquid exudates from passing through the backsheet.

The backsheet may comprise a woven or nonwoven material, polymeric films such as thermoplastic films, optionally apertured thermoplastic films, of compounds as described in the above, or composite materials such as a film-coated nonwoven material. The backsheet can be embossed and/or matte finished to provide a more clothlike appearance.

Backsheet materials may be executed unitary with the core, such as when a material layer providing the backsheet functionality is coated onto the backsheet side of a core.

According to the present invention, an absorbent core comprises absorbent materials. Within the present context, the term absorbent material refers to materials that can receive and retain liquids. In such absorbent materials, the liquid may be retained by a fibrous structure as inter-fibre or intra-fibre liquid. It may also be retained in absorbent gelling materials, often referred to as superabsorbent materials, such as - without limitation - polyacrylate or starch based, or it may be otherwise bound to materials of the absorbent structure.

Within the present context, the term "absorbent" and related terms refer to the ability of a material to receive liquids and to retain such liquids under certain conditions. Thus a material such as a cellulosic web can absorb aqueous liquids essentially by two mechanisms, namely retaining some liquid within the fibrous structure and by retaining liquids and possibly other materials dispersed in the liquid in interstitial voids between fibers. The terms "superabsorbent material", also referred to as "SAM", "superabsorbent polymer", also referred to as "SAP", "superabsorbent", "absorbent gelling material" or "AGM", "absorbent polymer material", mean partially cross-linked polymeric materials, often of the polyacrylic acid / acrylate type, which can absorb water whilst they are swelling to form a gel. Typically, such materials are particulate or in a fibrous form, in both forms being able to absorb at least 10 times their own weight, often more than 20 times or even more than 30 times their own weight when determined according to the centrifuge retention capacity (CRC) as described in more detail herein below. Such particulate SAP materials are broadly marketed such as from BASF, Germany, Nippon Shokubai, Japan, Evonik, Germany, LG, Korea, Danson, China, Satellite, China. Fibrous SAP is available e.g. from Technical Absorbent Ltd, UK. Biodegradable SAP materials such as being based on polyvinyl alcohols (PVA), polysaccharides, polyitaconic acid, or polypeptides, are available from e.g. Archer Daniels Midland, US, Exotech Bio Solutions, IL, TryEco, US, and Weyerhaeuser Company, US.

Liquids as may be suitably absorbed by the present invention are generally aqueous liquids, such as bodily exudates such as urine, menses, low viscosity faeces, blood, etc..

In a particularly preferred execution, the absorbent material in the absorbent core may be provided as an absorbent web material.

Typically, suitable absorbent webs exhibit saline Centrifuge Retention Capacity (CRC) capacities in excess of 2 ml / g of web material, often more than 4 ml/ g, more than 15 ml / g or even exceeding 30ml / g, as determined by the method as described herein below. Preferably, the material exhibits a folded absorbent capacity factor of at least 2 ml/g material, or more than 5 ml/g material, even more than 10 ml / g or even more than 15 ml/g material, when tested according to the Folded Capacity Test as described below. Accordingly, the material exhibits a basis capacity resulting from the core capacity factor and the material basis weight, which should however not be too high as the space of the body clefts is often limited and too high absorbency resulting in increased thickness may become uncomfortable. Thus, often a maximum folded thickness (i.e. the sum of thickness of both side portions) after swelling of less than 20 mm is preferred or even less than 15 mm or even less than 10 mm, is preferred.

Preferably, webs particularly useful for being used in the present invention exhibit a bending stiffness of less than 400 N/m, often less than 350 N/m or even less than 300 N/m, when tested in either longitudinal or cross-direction according to the stiffness test as described in detail in WO2011041352A1, to which for this test express reference is made. For certain applications, a too low stiffness might be perceived by the user to be too "flimsy" or it might reduce the ease of handling or application. Then the bending stiffness is preferably more than about 200 N/m or even more than about 275 N/m. Typically, an absorbent web suitable for the present invention exhibits a dry thickness of about 1 mm to about 10 mm.

Without any limitation, exemplary absorbent web materials may be airlaid fibrous webs, preferably comprising superabsorbent materials as referred to in the above, such as without limitation commercially available from Glatfelter, e.g. Glatfelter Falkenhagen, Germany, or Technical Absorbent Ltd, UK, comprising SAP fibres (SAF^{™}), or McAirlaid's Vliesstoffe, Germany.

Other preferred absorbent webs may be manufactured by combining superabsorbent particles with an open porous web and ultrasonically consolidating the web, such as described in WO2014/001487 or WO2014/001488. Such webs may be particularly suitable, when produced in-line in or via a buffer system in an upstream processing step.

Absorbent cores according to the present invention shall fit well into the overall article size requirements, optionally allowing for a perimeter around at least a portion of the circumscribing edges of the absorbent article.

However, it is highly preferred that the absorbent core does not exhibit sharp edges such as may result from rectangularly shaped cores, but that they exhibit rounded front and rear edges, as may be made very suitably by a process as described in the co-pending application GB1720942.0 (unpublished, attorney docket number 0207-2010). Optionally, the absorbent cores may exhibit an "isthmus" type design, wherein the length of the folding line is shorter than the maximum overall longitudinal extension.

The fluid absorbing capacities for an absorbent core for an article according to the present invention can be determined as described in the test method section herein below. Typically the absorbent core provides all or at least most of the absorbency of the article, as described herein below. Whilst an absorbent core may exhibit a homogeneous basis capacity, i.e. its absorbency is the same along various lines across x-y-direction of the article, it may also - and often preferably - exhibit a capacity profile, e.g. varying basis capacity along the longitudinal center line.

An article according to the present invention may comprise components that ensure the integrity of the article at least in its pre-use and in-use configuration. Such connecting is achieved by other bonding means other than glue or adhesives, such as heat or fusion bonding, whereby energy, such as from heating, applying pressure, or applying sonic, preferably ultrasonic energy, melts or plasticizes at least a portion of the compounds such that may form a connecting bond.

For particular executions, it may be preferred that articles according to the present invention are wrapped, optionally each single article is single article wrapped. Optionally, the wrapping may be designed such that the wrapping of a fresh article may be used for disposal of a used article. Optionally, the wrapping may be adapted to be flushable or degradable.

Several articles may be wrapped by the same wrapping webs, such as when folded or unfolded articles are enveloped between wrapping webs, such as films. Thereby the articles may be positioned in a single lane arrangement, i.e. the articles are positioned in a longitudinal sequence.

Alternatively, the articles may be positioned in a multilane arrangement, with two or more articles being positioned cross-directionally adjacent or staggered.

Optionally, though not necessarily, the articles may be positioned with their longitudinal extension coinciding with the longitudinal extension of the wrapping web. Articles according to the present invention may be suitably packed in a clean and shape protecting packaging, be these articles wrapped or not. Without intending any limitation, such packaging forms may include packing single-wrapped folded, unfolded, or partly folded articles into a package, such as a carton package. Optionally, articles, especially partly folded articles may suitably arranged in a tube like packaging in an intermeshing or overlapping arrangement. Optionally, articles wrapped between continuous wrapping webs may be rolled up, optionally so as to result in an overall roll width that may match the dimensions of conventional toilet paper rolls, such as with regard to width of between 6 cm and 12 cm, and inner (roll core) diameter of between 3 cm and 6 cm. The outer roll diameter may range from 10 cm to 20 cm for private household applications, though it may be significantly larger for e.g. public toilet applications.

Further useful components may be one or more of additives, such as selected from the group consisting of:
- lotions or emollients that might enhance the properties of the topsheet surface;
- odor control agents or perfumes;
- other health or wellness enhancer as may comprise medicaments as may be included in the above mentioned topsheet applied motions or emollients or odor control agents;
- liquid transport control agents, such as ant-wicking means that may reduce or even eliminate liquid transfer at the edges of the article;
- fixation aid, such as connecting means like adhesives or mechanical connecting means (e.g. of the hook and/or loop type), optionally combined with a release cover layer, whereby these fixation aids may also be employed on the topsheet surface (e.g. in case of body adhesives);
- application and/or removal aids such as pouches created for ease of applications and or removal strings or bands for ease of removal;
- edge barriers webs as may connected to the wearer oriented surface of the article along the longitudinally extending periphery of the article and extending towards the longitudinal center line for about 10 to 20 % of the maximal width of the article, which may be applied as separate webs or by overfolding e.g. the backsheet material;
- edge barrier agents as may be applied to the articles at least along its longitudinally extending periphery, optionally its full periphery, which may be hydrophobizing agents like silicone sprays or waxes or the like.

Though it is a particular aspect of the present invention that the products exhibit particular properties for disposal via the sewage system, i.e. flushability and/or (bio-) degradability, for certain applications or for certain regions it might be desirable to adapt the articles according to the present invention with particular features allowing easy disposal over the solid waste system, such as may be landfilling or incineration.

Thus the materials preferably have no substances that are harmful to the environment and fully biodegrade, i.e. decompose preferably to CO₂ and water only.

Also, products according to the present invention may comprise or be combined with packaging that provides good protection for a fresh product, but which may be used as wrapping for easy disposal of a used product.

### Article Design

An article according to the present invention is constructed of materials and components as described in the above. Overall shape and dimensions are determined by specific application to fit well into the respective body cleft.

For the application as an interlabial pad, the article is generally flat, i.e. thickness that not necessarily needs to be the same over the full area, but is generally significantly smaller than the other extensions. Typically, the in-use configuration will be such that at least two side portions extend away from a longitudinally extending fold line. For particular applications, the side portions exhibit a symmetrical shape relative to the longitudinal fold line. The article or the side portions may be, but do not need to be symmetric along any cross-directionally extending line. The article for such an application may exhibit an oval, elliptic or egg-like rounded shape. The term "elliptic" refers to the shape of an ellipsis, i.e. overall rounded shape with the rounding transitioning from a smaller elliptical diameter to a larger elliptical diameter. Egg-shaped refers to a rounded structure with gradual transition of sections that may be locally approximated by inscribed circles or ellipses. Oval refers to a tetragon, such as a rectangle or trapeze with rounded corners connected by straight line sections at least for a corresponding pair of opposite lines. This includes a shape, where the straight line(s) of one or two opposing sections are essentially non-existing, e.g. if a rectangular shape is complemented by two half circles on opposite sections.

Also included are generally rounded shapes, with locally varying diameters however not exhibiting gradual transitions but wherein neighboring sections may exhibit an angle at the connecting point. The article may also exhibit an "isthmus" shape, wherein the article length along the longitudinal fold line is shorter than the maximum longitudinal extension of the article.

The maximum longitudinal extension for such an application may be for the side portions more than about 50 mm, often more than about 65 mm or even more than about 80 mm, but less than about 110 mm, often less than about 100 mm or even less than about 90 mm, and most preferably from about 80 mm to about 90 mm.

Accordingly, the length of the longitudinally extending fold line may be the same as the maximum longitudinal extension, or e.g. in case of an "isthmus" at least 2 mm, often more than 10 mm or even more than 15 mm, but preferably less than 25 mm, often less than 15 mm or even less than about 10 mm shorter than the maximum longitudinal extension.

For the application as a haemorrhoidal pad the same shape and dimensions as for an interlabial pad may be applied.

For the application as an axillary or underarm sweat pad the same shapes as for the other applications may be used, however, a shape that is asymmetric to the longitudinal fold line might be preferred, such as by having a larger and a smaller side portion. Further the side portions may exhibit a shape as described for the total articles in the above. The longitudinal extensions may be for a first (e.g. larger) side portion more than about 70 mm, often more than about 80 mm or even more than about 90 mm, but less than about 120 mm, often less than about 110 mm or even less than about 100 mm, and most preferably from about 90 mm to about 100 mm. The longitudinal extensions may be for a second (e.g. larger) side portion more than about 50 mm, often more than about 60 mm or even more than about 70 mm, but less than about 100 mm, often less than about 100 mm or even less than about 90 mm, and most preferably from about 75 mm to about 185 mm.

The longitudinal fold line connecting the first and the second portions may exhibit a fold line more than about 50 mm, often more than about 60 mm or even more than about 70 mm, but less than about 120 mm, often less than about 110 mm or even less than about 100 mm. The width extension, i.e. the maximum extension perpendicular to the longitudinal fold line for the article may exceed the maximum longitudinal extension and be more than about 85 mm, often more than about 100 mm or even more than about 110 mm whilst being preferably less than about 150 mm, often less than about 140 mm, or even less than about 130 mm, most preferably between about 120 mm and 130 mm. The first (e.g. larger) side portion may exhibit a width of more than about 50 mm, often more than about 60 mm or even more than about 65 mm whilst being preferably less than about 110 mm, often less than about 90 mm, or even less than about 80 mm, most preferably between about 50 mm and 60 mm. The second (e.g. smaller) side portion may exhibit a width of more than about 30 mm, often more than about 40 mm or even more than about 50 mm whilst being preferably less than about 90 mm, often less than about 75 mm, or even less than about 60 mm, most preferably between about 50 mm and 60 mm.

It should be noted that in case of a folded product the overall width can be determined by the sum of the widths of the two portions extending from the longitudinally extending fold line.

The article may have extension that is essentially identical with the absorbent core, e.g. when the article is cut out from a composite multilayer material with integral topsheet, backsheet and absorbency functionality. In such an execution, it might be preferable to have to apply a moisture transfer hindrance means to at least a portion of the circumference.

More typically, however, the article will comprise at least for a portion of its circumference, a perimeter comprising a topsheet and a backsheet but no absorbent material, such that the topsheet and the backsheet are connected to each other. Optionally, the connecting may be executed such that it also functions as a moisture barrier, such as may be achieved by adhesive, or other barrier lines, that may be a continuous line or a series of bond points that are arranged along a line or that form a bonding region, such as of a pattern of bond points.

Articles according to the present invention are preferably thin in their z-directional extension, typically less than about 10 mm, often less than about 5 mm, whereby the thickness corresponds to an unfolded article or to the thickness of one side portion.

An article may further comprise any of the further components as described in the above.

### Article properties

An article according to the present invention should preferably exhibit certain properties as may be described by certain parameters that can be ascertained by the respective test methods.

The absorbent capacity of an article according to the present invention may be described by the CRC of the article, based on 0.9% saline, though e.g. for application as menstrual pad with other liquids such as artificial menses, may result in different amounts. Preferably, articles according to the present invention also exhibit good performance when not being positioned in a flat configuration, but when being in a folded, inclined and pressurized configuration, as may very well be assessed by the "Folded Capacity Test" as described herein below. The "Folded Drip Off Capacities" of the article may be adapted to the intended application and range from 1 to 5 or even 10 ml e.g. for an article for "drip incontinence" or a "haemorrhoidal pad". For e.g. light incontinence, a sweat pad, or an interlabial pad as a menstrual pad, capacities may range from 5 to 10 or even 20 ml, or for moderate incontinence even to 50 ml or above.

In order to minimize the risk of soiling of worn articles of the wearer, such as panties, the article should exhibit no or low side edge leakage as can be determined according to the "Edge Wetting Test" as described herein below.

For certain applications maximum surface dryness may be desired, e.g. for an application as underarm sweat pad. For certain applications, e.g. interlabial pad application, a balanced surface dryness as may be determined by the "Post Drip Off Capacity Test Rewet Test" as described herein below may be desired so as to not overly dry out the sensitive tissue. Apart from not negatively impacting the skin condition by the balanced surface dryness, articles according to the present invention shall be non-irritating to the skin, by exhibiting good softness, compressibility and bending stiffness, but by also not exposing any harmful or irritating substances.

For certain applications, articles according to the present invention shall preferably be adapted to be flushed away through the toilet. For most toilet and sewage treatment systems the articles will satisfy the flushability criterion because of their size such that they are not clogging the sewage systems and may be removed by sieving or other mechanical separation from the waste water. Depending on the particular system, it may be preferred if the articles are floating, and thus are more readily transferred to a sewage treatment plant, or if they are sedimenting that may ease separation. However, for certain systems it may be preferable that the articles disintegrate upon exposure to excess water. This may be achieved by materials or compounds as described in the above.

Further, articles according to the present invention may be disposed through various waste channels that allow controlled incineration or less preferred land-filling and the articles shall not impose any threat to the environment along these systems. However to allow even broader use with almost unrestricted selection of ways of disposal, articles according to the present invention exhibit degradability, such as upon exposure to UV- or sun-light, but even more preferably bio-degradability.

### Article manufacturing

An article according to the present invention may be manufactured by combining at least topsheet and backsheet with an absorbent core. The materials may be provided as web materials or may be formed just prior to the combining with the other webs, such as when superabsorbent particles are added to a web material, or if a web material is rendered liquid impermeable by adding a coating. The materials may be connected by known methods such as by application of glues or adhesives or by melt fusion bonding.

In the preferred execution of preformed absorbent webs, these may be enveloped between topsheet and backsheet and the articles may be cut out from this composite. However, this may create substantial trim, which may be acceptable for simple core materials that are easily recyclable or - though less preferred - for cheap core materials, which may then be discarded.

Alternatively, the core webs may be cut prior to being enveloped between topsheet and backsheet. However, as simple absorbent core shapes, like a rectangular shape, are less preferred from a user acceptance point of view, the core cutting should create rounded edges, at least at the front and rear edges. Apart from conventional approaches that still create trim of the core material that might be recycled or disposed, a particularly preferred approach provides layered core structures in a trim-free approach

### EXEMPLARY EXECUTIONS

In order to better explain the principles of the present invention, reference is made to the figures, wherein certain exemplary but in no way limiting executions are schematically depicted. It should be noted, that various features and elements may be used as such but also in combination with other features or elements even though no explicit reference is made.

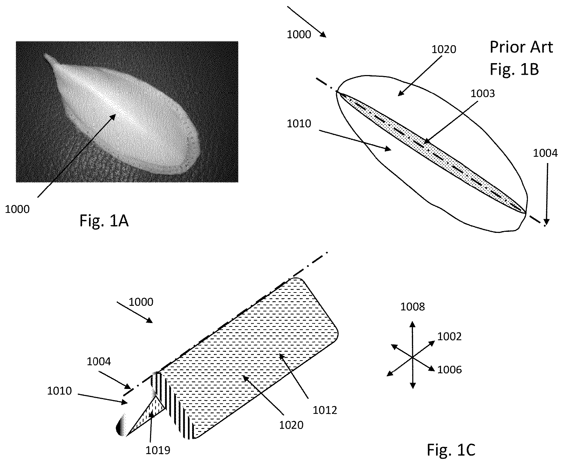

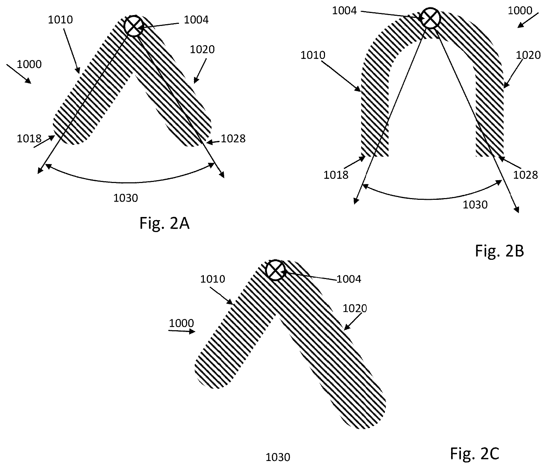

Fig. 1 shows an article useful as an interlabial pad, with Fig. 1A to 1C showing a perspective view of an article 1000 in Cartesian coordinates with a longitudinal direction 1002 and a longitudinally extending fold line 1004 in a folding ridge region 1003, a width direction 1006 and a thickness direction 1008. The article further comprises two side regions 1010 an 1020 extending away from the fold line 1004, as shown in a cross-sectional view in Fig. 2 A to F, in various executions, respectively. In Fig. 2A, the article is shown in a folded configuration such that the side portions 1010, 1020 are connected at the fold line 1004 to form an angle 1030 between them. In Fig. 2B, the article does not exhibit a sharp fold line but rather a more gradual bending which may be approximated by the shape of an inverted U. Then, the fold line 1004 corresponds to the upper ridge portion and the folding angle 1030 is defined by this fold line and the end points of the side portions 1018 and 1028, respectively. As indicated in Fig. 2C, the side portions 1010 and 1020 are not necessarily of the same shape or size.

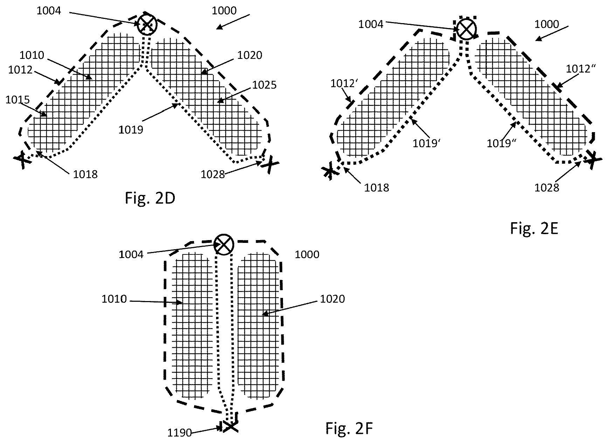

In Fig. 2D, an article 1000 is presented with side portions 1010 and 1020, respectively, and a longitudinal fold line 1004. Further, each of the side portions comprises an absorbent core 1015 and 1025 respectively. A single topsheet 1012 covers both user oriented surfaces of absorbent cores 1015 and 1025, respectively, which is further connected to a single backsheet 1019 at the end points of the side portions, 1018 and 1028, respectively, and in this exemplary execution at the fold line 1004.

In the execution as shown in Fig. 2E, the topsheet and backsheet is not unitary for both side portions, but at the fold line 1004 each a backsheet 1019' and 1019" and a topsheet 1012' and 1012" of the first and second side portion are connected, here shown in a butt seam mode, though this can also be executed as an overlapping arrangement with an overlap seam.

Further, Fig 2F depicts schematically an arrangement of fully overfolded side portions, such that the fold angle approaches 0°. In such an execution, the end points of the side portions may be connected by a connection 1190 to each other close to the or at the end portion at edge or perimeter of the article, such as by a glue or melt fusion bond point or line.

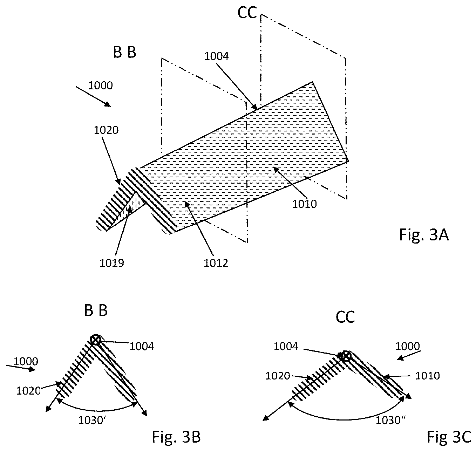

A particular execution according to the present invention is schematically depicted in Fig. 3 relating to an absorbent article in a pre-use configuration as may be fully overfolded towards a first longitudinal end portion as depicted in Fig. 2F (not shown) with an increasing folding angle 1030' towards the opposite end portion, 1030", respectively.

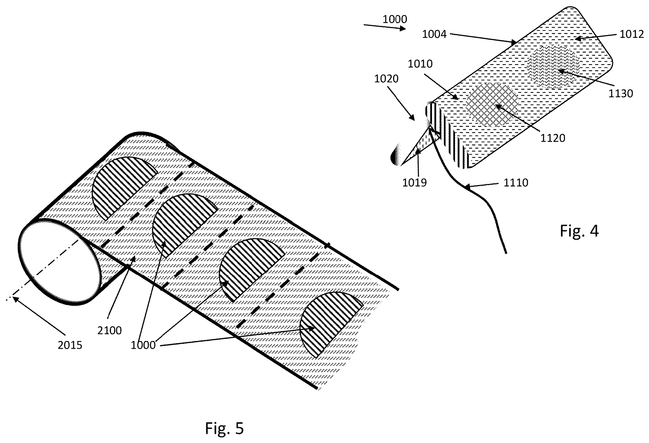

Fig. 4 shows schematically further elements as may be embodied in an article according to the present invention, comprising a removal aid in the form of a string 1110. Further, the article may comprise a surface topsheet 1012 comprising an emollient or lotion 1130 or a fixation aid applied to the surface 1120. In a particular variant thereof, the additive like the lotion may be applied by a user, whereby s/he may selects among different additives e.g. according to the purpose like skin friendliness, odor reduction or masking, cleaning and so on.

Fig. 5 depicts schematically an execution wherein articles according to the present invention are wrapped in wrapping material 2100 and wound up on a roll around a roll axis 2015. As shown, the longitudinal orientation of the articles, here shown in their pre-use configuration being fully overfolded articles, is perpendicular to the unwinding direction of the roll.

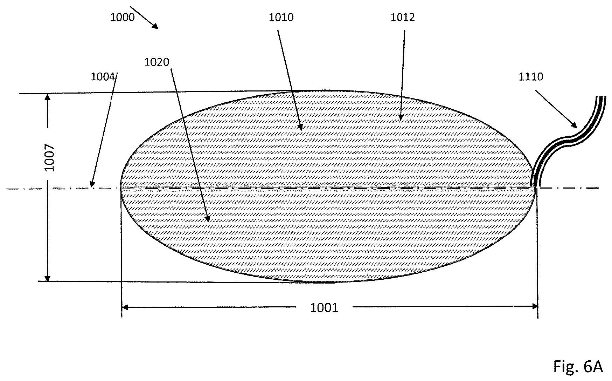

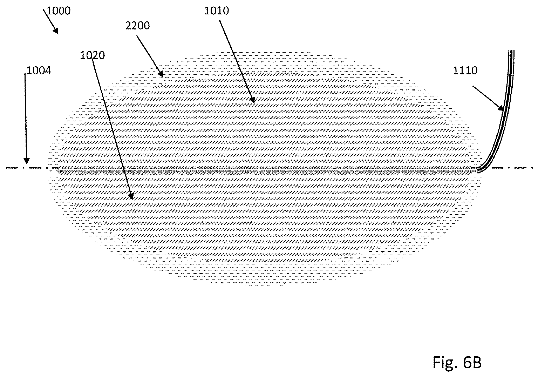

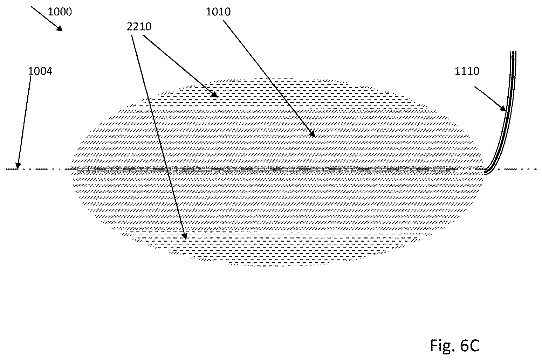

Further details of an article are depicted in Fig. 6 A exhibiting a maximum length 1001, a maximum width 1007, and the longitudinal fold line 1004. Also indicated is a removal string 1110. In Fig. 6B and C executions are depicted, where the absorbent pad comprises a periphery 2200, which may be bonded adhesively or by melt fusion bonding. Optionally, the periphery may be treated with wicking limiting agents as described in the above. Alternatively only a portion of the periphery may be treated, such as along the longitudinally extending side edges 2210, see Fig. 6C.

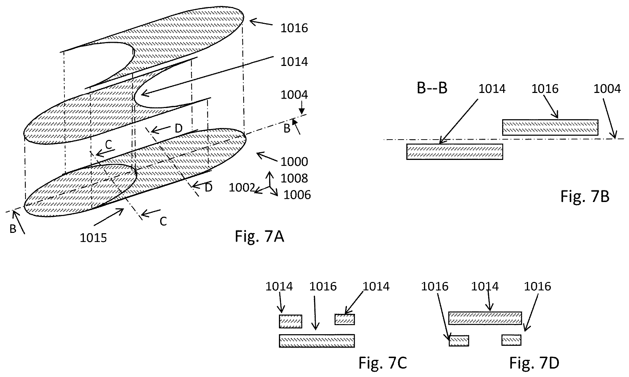

Fig. 7 relates to a particularly preferred execution wherein the absorbent core exhibits rounded longitudinal end portions and essentially straight side edges, with Fig 7A depicting schematically an exploded and combined perspective view of an absorbent core 1015 comprising two sub-layers of absorbent web material 1014, 1016, that are z-directionally partly overlapping and longitudinally offset, with Fig. 7B depicting schematically a longitudinal cross-sectional view and Fig. 7C and 7D cross-sectional views in the cross-direction. Such an execution is particularly beneficial as it allows rounded end portions that may be manufactured essentially trim-free. As can be seen in Fig. 7A, such a core 1015 may be suitably folded along the longitudinal fold line 1004, forming a core with 1^{st} and 2^{nd} side portions of an article.

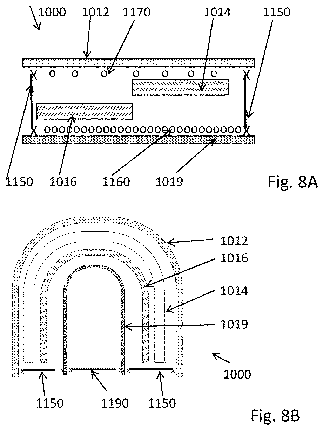

In Fig. 8A and B an article 1000 comprising a core made of the two sublayers 1014, 1016 enveloped between a topsheet 1012 and a backsheet 1019. The core may be connected to the backsheet or topsheet as indicated by a glue coating or spray 1160 and/or by a bonding point pattern 1170. In the perimeter of the article, the topsheet 1012 and backsheet 1019 may be connected to each other, as indicated by connection 1150. Fig. 8B depicts such an article in a folded configuration, further comprising a connecting 1190 between the overfolded side portions.

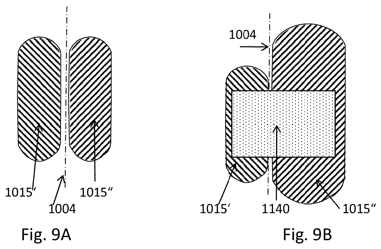

Fig. 9A depicts a further arrangement of cores for an absorbent article according to the present invention, wherein two separate cores 1015' and 1015", each of which may comprise sub-layers of core material as explained in the context of Fig. 7, are positioned parallel to the longitudinal fold line 1004, thusly each one forming a side portion. Such side portions not necessarily have to have the same size, as shown in Fig. 9B, and in Fig. 9B such cores for the first and second side portion may be bridged across the fold line by a further material, such as a further absorbent patch 1140.

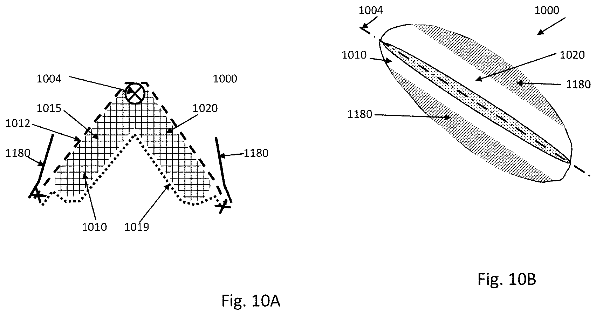

Fig. 10 A and B depict an article comprising edge barriers webs 1180 as may be connected to the wearer oriented surface of the article along the longitudinally extending periphery of the article, extending towards the longitudinal center line for about 10 to 20 % of the maximal width of the article.

### Reference sample Sweat Pad (SP-R):

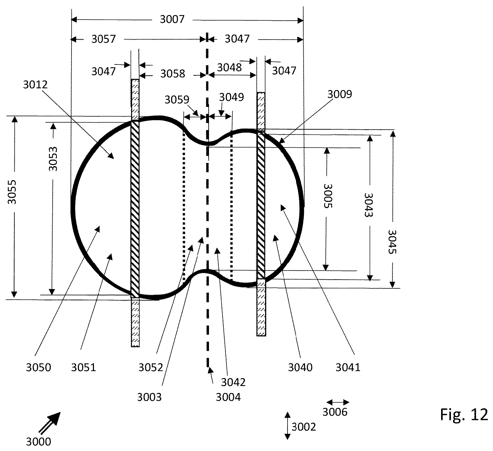

In Fig. 12, reference is made to an absorbent article 3000 as commercially available from HialyPack, Changxi Hu as "Absorb Sweat Underarm Pads", e.g. via e-bay ^{™}. The pad 3000 exhibits an average total pad weight of about 1.6 g and a shape as depicted in Fig. 12 with two wings or side portions of uneven size connected by fold region 3003 with a fold line 3004, which - in Cartesian coordinates with a width direction 3006 and a length direction 3002 - exhibits a fold line length 3005 of 65 mm, and which separates the pad into a first (3040 - here shown as smaller) and a second (3050 - here shown as larger) side portion. The first side portion 3040 exhibits a maximum width extension 3047 from the fold line 3004 laterally outwardly of 55 mmm and a maximum length 3045 of 80 mm. The second side portion 3050 exhibits a maximum width extension 3057 from the fold line 3004 laterally outwardly of 70 mm and a maximum length 3055 of 95 mm, such that the pad exhibits a maximum width over both side portions 3007 of 125 mm

Over its full area, the pad is made up of a conventional, spunbonded nonwoven of approximately 17 g/m² as topsheet 3012 oriented towards a wearer during use and facing the viewer in Fig. 12 and a conventional polyethylene film of approximately 20 g/m² as opposite backsheet. Between the topsheet and the backsheet a layer of cellulose fluff at approximately 57 g/m² and approximately 0.44 g fluff per pad, is positioned as absorbent core, also extending over the full area, and exhibiting a first (smaller) side portion core 3041 and a second (larger) side portion core 3051. The backsheet, the topsheet and the fluff layer, where present, are bonded by adhesive spray and further edge bonded by embossing at a 5 mm rim 3009 along the periphery of the pad. On the backsheet side, a release paper is covering a garment adhesive layer, extending towards the outer edge of the pad, except for the fold region 3003 along the longitudinal fold line 3004, with a first fold region 3042 extending at a first side portion fold region width 3049 of 10 mm, and a second fold region 3052, extending at a second side portion fold region width 3059 of also 10 mm.

The asymmetric drip off capacity test as described herein below at the low pressure was executed at a load bar distance from the fold line 3048 (first, small side portion) and 3058 (second, large side portion) of 25 mm and 33 mm respectively and at a load length 3043 (first, small side portion) and 3053 (second, large side portion) of 70 mm and 85 mm, respectively, resulting in a load bar load of 140 g (first, small side portion) and 175 g (second, large side portion). The first drips were noticed at 4.665 ml and 2.634 ml from the second, large and first, small pad side portion, respectively, resulting in an average load factor of 16.0x of the absorbent core (here cellulose) weight, before the drips started to leak. Loading each of the sides portions with 80% of the thusly determined core capacity factor resulted in a rewet of 1.38 g.

### Test sample Sweat Pad-1 (SP-1)

A sweat pad was constructed, that exhibited the same outer (i.e. topsheet and backsheet) dimensions as sample SP-R. However, instead of the cellulosic core, a pre-manufactured air-laid nonwoven web from Technical Absorbents, Grimsby, UK, under the trade designation SAF^{™} , type 0076A235100, comprising fluff pulp, bicomponent polyethylene/polypropylene fibers and a nonwoven web in addition to superabsorbent fibers. It exhibited a basis weight of 76 g/m² and a thickness of 0.8 mm. At the pad periphery, it was cut at a size 5 mm smaller than the topsheet/backsheet, where the topsheet, a conventional 17 g/m² spunbondend polypropylene web, and backsheet, a conventional polyethylene film of approx. 20 g/m², were connected to each other by thermal bond. Further, the fold regions were left free of the material for about 10 mm on each side portion. Thus, approx. 438 mg of absorbent material were placed in the second (larger) side portion, and about 268 mg of material in the first (smaller) side portion. The pad showed a good softness. Submitting the side portions to the asymmetric low pressure core Drip Off Capacity Test resulted in a drip off test capacity of approximately 11,634 ml or drip off core capacity factor of 16x.

### Test sample Sweat Pad-2 (SP-2)

A further test sample was prepared as SP-1, except for the absorbent core. For this, superabsorbent material, commercially available from Ekotec Group, Haan, Germany, under the designation EK-XEN 67, was sieved to remove coarse particles of more than approximately 500 µm. 1 g of this was distributed at a basis weight of approximately 130 g/m² between through-air bonded nonwoven webs STA1PBL30 of SHALAG, Israel, a through-air bonded web of a blend of bicomponent polyethylene/polypropylene and polypropylene fibers at 30 g/m². The pad showed a very good softness. In the low pressure drip test, the large pad side portion received 23.238 ml of test fluid, or a load factor of 23x, before the side seal failed, but still no dripping had occurred.

### Test Sample Interlabial Pad (ILP-1)

An absorbent pad was prepared, comprising a topsheet and a backsheet, and exhibiting an essentially elliptical shape with a longer axis of 85 mm and a shorter axis of 45 mm (See Fig. 6A), which were connected along their periphery by thermofusion bonds. The topsheet is conventional spunbonded polypropylene non-woven of approximately 17 g/m², the backsheet is a conventional so called "textile backsheet" of approximately 22 g/m², comprising a polyethylene film and an outwardly oriented layer of polypropylene fibers Between the top- and the backsheet, an absorbent core was positioned at a size that provides space for a 5 mm bonding region for connecting the top- and the backsheet, i.e. as a ellipsis of 75 mm by 35 mm. The core material was an airlaid fabric with fluff-pulp, BiCo fibers and superabsorbent material fibers enveloped between non-woven layers of a total basis weight of 240 g/m² and commercially available from Technical Absorbents, Grimsby, UK, under the designation Airlaid 2717. It exhibits a nominal caliper of 1.3 mm and a nominal CRC capacity of 21 ml of 0.9 % saline per gram. The pad exhibited an overall weight of 704 mg. In a free swell test, the thickness of the absorbent material increases by approximately 2.5 mm. The pad is longitudinally folded and the fold affixed by a thermofusion bond point in the periphery, at a distance approximately 1 cm away from the extremity of the longitudinal fold line when projected to the fold line. Prior to testing, this bond point is carefully opened to allow positioning of the test specimen on the test specimen support. During the drip test it is watched that the opening is not leading to premature dripping,

The pad was submitted to the symmetric low pressure drip test as described herein below, whereby the test liquid was applied dropwise to the side portions, whereby upon a load of approximately 0.75 ml the respective other side portion was loaded alternatingly until the first drip was released from the first side, upon which the other side portion was loaded (in this case by additional 0.5 ml) until a drop was also released there. Overall nine full loads plus the partial last loads were discharged per side portion. After three loads on each side, the distance of the load bars was carefully increased to compensate for the thickness increase, by approximately 0.8 mm (as determined by the count of the adjustment screws and confirmed by measurement). This was repeated after three more loads.

The total pad was carefully removed from the test stand and the weight increase determined to 14.967 ml per pad or 20.9 ml of 0.9% saline per g of core material.

### Test Sample Interlabial Pad (ILP-2a to 2c)

Further test samples were prepared as per the design of ILP-1, except for the type of core material, namely an airlaid fabric with fluff-pulp, BiCo fibers and superabsorbent material fibers commercially available from Technical Absorbents, Grimsby, UK, under the designation Airlaid 2351, which exhibits a nominal basis weight of 76 g /m², caliper of 0.8 mm and a capacity of 24 ml of 0.9 % saline per gram. The three samples were prepared by using a single layer (sample ILP-2a), a double layer (sample ILP-2b) and a triple layer (sample ILP-2c) of this material and submitted to the low pressure drip test as described herein by applying approximately 1ml per side in 30 seconds, waiting 30 seconds and loading the second side, and so on, until release of the first drop. The absorbent core of the samples had a weight 138 mg, 276 mg and 414 mg, respectively, and after the release of the first drop, the samples exhibited a total weight of 2440 mg, 4123mg, and 5458 mg, respectively, or a drip off capacity factor of 15.7 g/g, 13.4 g/g, and 11.8 g/g, respectively.

This indicates that under the conditions of the test the core efficiency decreases with increased basis weight of absorbent.

### Test Sample Interlabial Pad (ILP-3)

A further test sample was prepared as per the design of ILP-2b, except that instead of a poly for the backsheet material a hydrophobic spunbonded NW of about 19 g/m² was employed, which was on each side folded around the longitudinally extending portions of the core and topsheet for about 10 mm, i.e. overlapping the core at its widest portion about 5 mm. The absorbent core exhibited a weight of 282 mg, the total pad of 435 mg. The first drop was released at a total weight of 5331 mg, or at a specific drip off capacity of 17.4 g/g.

### Test sample Interlabial pad (ILP-4)

An even further test sample was prepared with an absorbent member with dimensions and topsheet and backsheet material according to ILP-1 and -2. For the absorbent core, superabsorbent particles were used as per SP-2 at 600 mg per pad and placed between two layers of embedding material ATB NW Vortex 60 g/m² commercially available from Texsus s.p.a., Italy, with the skin layer positioned "outwardly" i.e. to the topsheet and the backsheet, respectively. The pad had a total weight of 982 mg and exhibited very good softness. No drop was released until the failure of a side closure due to strong swelling at a total weight of 12113 mg, corresponding to a specific core drip off capacity factor of 13.2 g/g.

### TEST METHODS

All tests should be carried out under standard laboratory conditions, such as more specified in EDANA NWSP 003.0.R0 (15).

Liquid Absorption capacity can be determined and described by several parameters and respective test methods.

Centrifuge retention capacity (CRC) is a well-established method. In EDANA NWSP 241.0.R2, a method is described for determining the capacity of superabsorbent material. For determining the Centrifuge retention capacity of absorbent web material, this standard test can be modified in that it is applied on a 40 mm x 20 mm cut-out sample of a region of the absorbent web materials. Each sample should then be handled and measured as specified in the EDANA method, e.g. stored in closed container and conditioned prior to measurement

Simplified approximation of absorbent capacities for mixtures of cellulose and superabsorbent materials can be made by taking the CRC of the SAP raw material, that is typically known for such materials, and assuming a value of 4 ml/g for cellulose fluff.

### "Folded Capacity Test"

Application: The test specimen for the present test may be absorbent devices as well as absorbent members or cores that are intended to be used for or comprised in such devices. In the first case, referred to as "Folded Product Capacity Test", the absorbent devices products may be used as such or may be modified to be testable in the present test such as by allowing to take a foldable, but essentially flat configuration, e.g. by carefully removing the connection between the outward parts of the side portions without damaging the integrity of the pad. For the second case, referred to as "Folded Core Capacity Test", the absorbent members or cores are preferably tested between surface layers, such as a topsheet and a backsheet material or surface layer, that may be of the type as intended for forming the absorbent device. In case of testing absorbent members without such defined topsheet or backsheet material, the absorbent members should be positioned between a standard, conventional topsheet materials (hydrophilic spunbonded nonwoven of 17 +/- 2 g/m² basis weight with monofilaments of between 1 and 3 dTex), and standard conventional backsheet materials (polyethylene or polypropylene films at between 10 and 20 g/m² basis weight). The topsheet and backsheet should not extend beyond the perimeter of the absorbent member by more than 1 cm. No additional glues should be applied to the test specimen, although the margins of the topsheet and backsheet may be attached to each other, such as by heat or ultrasonic bonding.

Principle: The test aims at reflecting in-use conditions for a folded product in a body cleft, where a liquid is primarily transferred to the absorbent device along the contact surface between the wearer and the device, which is within the side portions of the device rather than along the upper ridge or longitudinal fold line.

The Folded Capacity Test includes various subtests that may be executed all together or individually on different specimen of the same product or core design. These subtests include determining
- Drip Off Capacity, which may be expressed in "ml per pad or core" or in as a "drip-off Capacity factor" as the ratio of the absorbed liquid to the weight of the absorbent material or core;
- edge wetting
- Post Drip Off Capacity Test Rewet Test
- Rewet Test at Design Capacity, as may be 80% of the Drip Off Capacity, which can be executed at a
- low pressure condition at 0.981 kPa (also expressed as 10 g/cm² load); or a
- high pressure condition at 3.92 kPa (also expressed as 40 g/cm² load),
and each for an absorbent pad or product or an absorbent core.

When reporting results, it should be made clear, which subtest or condition has been executed, e.g. by quoting "Folded Core Low Pressure Drip-off Capacity".

The following is described for application to devices which are essentially flat in a pad form, such as underarm sweat pads, and/or symmetric to a longitudinal fold line, such as to interlabial products, and which further exhibit an overall product length of between 60 and 130 mm, and an overall pad with of between 15 mm and 50 mm folded, resp. 30 mm to 100 mm flattened out. Further, they typically exhibit an absorbent capacity in saline or design capacity, respectively, of between 1 ml and 20 ml. Adaptation of the test to other products is described in a later portion of the description. The term "test specimen" refers to the absorbent article or to the absorbent member of such an article, as specified in the specific test.

Generally, the test should be set up such that it is protected from excessive air drafts, that might cause drying of a loaded during waiting periods.

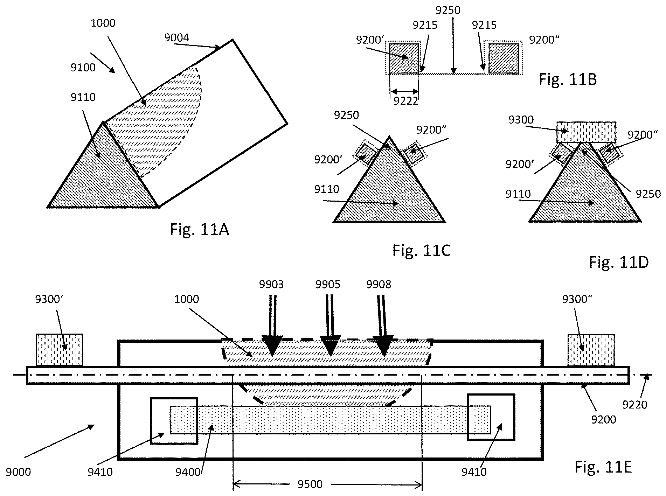

In Fig. 11 A to E a test stand 9000 is depicted in more detail.

A test specimen support 9100 is adapted to allow positioning of a test specimen 1000 in a non-flat configuration with its fold line 1004 over and aligned with the upper ridge 9004 of the test specimen support. It has a shape such that the width/height cross-section forms an equilateral triangle which has a side length of at least the folded test specimen width, preferably more, e.g. of 40 mm. The length should be at least the test specimen length, preferably more, e.g. 120 mm. The edges should be sharp and exhibit a radius of not more than 1mm. The material can be any liquid insensitive material, e.g. Perspex, PVC, metal or waterproof treated wood.

Two load bars 9200', 9200" are made from the same or different liquid insensitive material and are adapted to be positioned over the test specimen 1000, when resting on the test specimen support and hanging down the sides from the fold line 1004. The longitudinal center lines 9220 of the load bars extend parallel to the fold line 1004. The load bars 9200 exhibit a well-defined cross-sectional shape in the width - height dimensions, preferably with a side length of 5 mm that is adapted to rest flat on the test specimen support, such as a square 9210 or triangular cross-section, and have an overall length exceeding the length of the test specimen support 9100, such as by 60 mm or more, such they can protrude approximately 30 mm or more on each longitudinal end side to allow placing of the load weights thereon or hanging them thereon. The two load bars are connected to each other by a connection 9250 allowing a precise distancing of the load bars from each other and relative to the support. The connection may be a flexible one, such as an adhesive tape, or an adjustable fixed distance mounting. In order to avoid compression of the test pad by the connection, the connection is laterally outside of the support by an essentially horizontal connection. In particular for absorbing materials that exhibit high swelling in the thickness direction, the connection of the load bars may be executed such that the distance of the load bars may be readily and precisely adjusted, optionally even during testing. Alternatively, the load bars may be suspended from a gibbet that may extend essentially parallel and above the upper edge of the test specimen support. Care should be taken that the connection 9250 is executed such that the load bars are parallel to each other and to the support, and do not prevent the flat positioning of the load bars on the test specimen 1000.

The load bars are loaded by load weights 9300', 9300", which may be placed on the projecting portions of the load bards, or which may be suspended from these portions. They are of a type for which the weight can be precisely adjusted, preferably to one gram accuracy, e.g. a cubical or rectangular container, that can be placed on the protruding portions of the load bars and can be filled with appropriate weight adjustments, e.g. lead, lead balls, sand, water, and the like. A preferred execution uses magnetic spheres of 5 mm or 6 mm diameter, optionally in combination with other magnetic weights, so as allow easy adjustment or suspension. The load weights are adjusted to create the predetermined test weight as the sum of the weight of the test bars, the load weight container and the load weight.

Generally, 0.9 % saline solution can be used as test fluid, though other fluids may be applied such as synthetic menses according to EDANA NWSP 350.1.R1, therein applicable for tampons. Optionally, e.g. to allow for better visual observation, coloring (e.g. food coloring) may be added, however, the surface tension of the fluid should not be impacted by this.

The test specimen should be conditioned to the standard conditions in an air tight pack so as to avoid any potential moisture pick up from the air.

For executing the Edge Wetting Test, absorbent edge wetting pick up paper 9400, e.g. Whatman filter paper grade 3 or equivalent, is cut to a strip of 1 cm width and a length shorter than the test specimen support, but longer than the maximum test specimen length. It is overfolded evenly along the longitudinal direction, and the longitudinal ends of the overfolding are fixed by a small piece of adhesive tape 9410. Before being attached to the test specimen support, it is carefully weighed, preferably to a mg reading. Then it is attached to both flanks of the test specimen support such that the open edges direct upwardly and form a pocket into which the lowermost edge of the test specimen fits.

An alternative set up for the Edge Wetting Test may be more suitable for the case that the backsheet material is not liquid impermeable and thusly may transmit some fluid. For this execution, a 5 mm wide strip of edge wetting filter paper is attached over its full lower longitudinal edge with an adhesive tape, that covers approximately half of the strip and the rest being positioned on the test specimen support, thusly forming a pocket between the pick-up paper and the surface of the test specimen support, into which the lower end of the end edge may be positioned. Care should be taken that all of the edge wetting pick-up paper is positioned below the load bar.

Certain parameter for the test execution depend on the particulars of the test specimen:
The distance of the load bars from the longitudinal fold line 1004 is adjusted by the length of the load bar connection 9250 such that the distance of the upper edge of the load bar to the fold line corresponds to half of the maximum width of the folded test specimen. Preferably, the length of the load bar connection can be adjusted so as to adapt to the test specimen of different size, or even to the increase in thickness of a test specimen during the loading, such as by using a screw-nut system that can be manipulated even during the testing procedure.

The load area is determined by the width of the load bars and the test specimen load length 9500 as the length of the test specimen along the longitudinal center line of the load bars.

In order to achieve the desired pressure to the normal of the surface of the pad on both flanks or side portions of the test specimen, the desired pressure is divided by the cosine of the test specimen support angle (i.e. of 60°, corresponding to a value of 0.5), multiplied with the load bar width (i.e. 5 mm) and the test specimen load length 9500.

Thus, to achieve a "high pressure" of 3.92 kPa (or 40 g/cm²), for a test specimen load length of 70 mm, a load weight of 280 g should be applied for each side, or a total of 560 g, made up of the weight of the load bars and two sets of symmetric weights on each side on the load bars. Accordingly, for a "low pressure" of a 25% of the "high pressure", the total load should be a total of 140 g - or 20 g per cm of load length

The sample weight is accurately determined, e.g. on a mg reading scale, and the sample is positioned with its longitudinal fold line over the test specimen support, such that the side portions extend laterally downwardly, symmetrically to the longitudinal symmetry line, if such exists.

The load bars are applied so as to be aligned with their upper edge with the mid-section line of the test specimen, and the load weight is applied.

All test options are executed by preparing a drip pipette and applying approximately 1 ml of fluid dropwise to the test specimen with drops of 100 +/- 50 ml per drop within approximately 30 secs. The drops are applied such that they hit the test specimen from a falling height of not more than about 2 cm and just upwardly of the load bar. For an asymmetric test specimen, the drops are applied to one side portion only, and on each side spread longitudinally at 25%, 50% and 75% of the total load length, as indicate by arrows 9903, 9905 and 9908, respectively, in Fig. 11E, with about the double amount at 50% compared to 25% and 75%. After 30 seconds, during which the first ml is applied and a waiting period of 30 secs, another ml is dropwise added in course of the next 30 secs, and the procedure is repeated until the first drop falls off the pad. Then the addition of liquid is stopped. After a further waiting time of 1 min, the loaded pad is accurately weighed to a mg reading and the "drip off capacity" for a given pressure for a first side portion is recorded. Then the test is repeated with a fresh pad and the opposite side portion. The Drip Off Capacity may be expressed on a per pad basis (ml per pad) or on a core weight basis as Drip Off Capacity Factor in ml per gram of core material. In a variant of this test particularly suited for symmetrical test specimen and/or if there is good fluid transfer between the side portions, the first and second side portions of a pad are loaded alternatingly after the respective waiting period. If a pad exhibits good lateral fluid distribution properties, the ratio of the single side portion loading test and the dual side portion loading test approaches 1.

After the Drip Off Test, the Post Drip Off Capacity Test Rewet Test may be performed. To this end, a flat pad rewet weight is prepared to create for the particular surface area of the core a low (0.981 kPa) or high (3.02 kPa) pressure. For example, for an ellipsoid core with a long half axis of 40 mm and s short half axis of 20 mm, the area is 25.13 cm² and the respective low pressure (0,981 kPa) load is 251 g, or 1 kg for the high pressure (3.92 kPa). Further, filter paper (Whatman #3, 6 µm, or equivalent) is prepared at a size larger than the unfolded pad and accurately weighed to a mg reading. Within 1 min after the weighing, the pad is placed with its loaded surface up on a flat surface, covered by a flat plate (e.g. made of Perspex) and the filter paper is positioned between the pad and the respective rewet weight. After a waiting period of 1 minute, the filter paper is reweighed and the flat pad rewet determined on a pad basis.

A core rewet test may also be performed when loading the pad with 80% of the drip-off capacity and measuring the rewet as described hereinabove.

As an alternative to the filter paper, a pre-conditioned collagen film as purchased from NATURIN GmbH, Weinhein, Germany, under the designation of COFFI and at a basis weight of about 28g/m² may be employed and the result reported as such.

For executing the Edge Wetting Test, a further test specimen of the same type as for the Drip off capacity test is prepared in the same way and positioned such that its lowest edge fits into the pocket of the edge wetting pick up paper. Then the test fluid is applied as in the above until an amount of 80% of the Drip off capacity is reached without any waiting time.

After a waiting time of 3 minutes, the test specimen is removed and accurately weighed to confirm the actual fluid load, as well as the edge wetting pick up paper, from which the edge wetting amount is calculated.

For stress test conditions, the edge wetting test may be repeated with an increased amount of test fluid applied thereto, such as 85%, 90%, or even 95% of the drip off capacity, and the respective results should be monitored and clearly marked as such.

Considerations for particular test specimen shapes:
For test specimen exhibiting a pre-folded configuration a fixation means should be carefully opened so as to allow flat positioning on the test specimen support of the test stand.

For test specimens which are asymmetric relative to the longitudinal fold line, each side portions shall be evaluated separately and reported accordingly.

For test specimens which are asymmetric relative to a cross-directional fold line, the positioning of the load bars shall be calculated for the biggest cross-directional extension.

For test specimens which have one or more indentations along the longitudinally extending margin, such as exhibiting a so called butterfly shape, the positioning of the load bars shall be calculated for the biggest cross-directional extension. If the load bars extend over such an indentation, only the length where they rest on the test specimen are counted for the test specimen load length.

For a test specimen, which comprises a bulge or hump or downward fold along the longitudinal fold line, the test set up may be adapted such that the flanks, on which the test specimen rests on the support, are removable such that a longitudinal gap along the upper ridge of the support is created, into which the bulge or hump or downward fold can be fitted.

Thickness can be determined according to EDANA method NWSP 120.6.R0 (15).

Resistance to linting can be determined according to EDANA NWSP 160.1.R0 (15) / ISO 9073-10. Flushability can be determined by adapting the "Guidelines document for assessing the flushability of non-woven disposable products" as issued jointly by INDA and EDANA, 2013, and respective updates to the articles according to the present invention, which typically exhibit a smaller size than the wipes for which the EDANA / INDA guidelines are being developed..

Biodegradability refers in the present context to a materials or compounds such as a polymer, layer, film, particle, or fiber that are capable of being degraded completely or substantially completely into carbon dioxide, water, biomass and inorganic materials by or in the presence of microorganisms. The biodegradation potential can be estimated by measuring carbon dioxide evolution and dissolved organic carbon removal from a medium containing the substance being tested as the sole carbon and energy source and a dilute bacterial inoculum obtained from the supernatant of homogenized activated sludge. See Larson, "Estimation of Biodegradation Potential of Xenobiotic Organic Chemicals," Applied and Environmental Microbiology, Volume 38 (1979), pages 1153-61, which describes a suitable method for estimating biodegradability. Further determination of bio-degradability can be made according to ISO 17088 / EN 13432 / EN 14995 for compostable plastics and products made of compostable plastic materials. It addresses such aspects as biodegradation and disintegration during composting as well as negative effects on the composting process or the quality of resulting compost. It strongly relates to the ASTM standard D6400, defining the labelling of plastics that are originally designed to be aerobically composted. This standard covers plastics as well as products made from plastics that are designed to be composted in municipal and industrial facilities. Even further, ASTM D5338 / ISO 14855 provide test methods for the determination of plastics aerobic biodegradation under controlled composting conditions. This standard defines a test method which helps to determine the degree and rate of aerobic biodegradation of plastics in a controlled composting environment, that is, under laboratory conditions.

### Caliper

Express reference is made to US8314286, wherein a test method using a footed dial (thickness) gauge, Model No. ADPII16, available from B. C. Ames Company, of Waltham, Mass., with a 29.6 mm diameter foot with stand, 56.7 gm. deadweight accurate to 0.0254 mm is describe in full detail. Equivalent methods may be applied.

### Gurley Stiffness

Gurley stiffness is one of many indices of softness, and described in full detail in US8314286, to which express reference is made with regard to this method. Gurley stiffness measures the bendability or flexibility of absorbent materials. The lower the Gurley stiffness value, the more flexible the material. The Gurley stiffness values are measured using a Gurley Stiffness Tester (Model No. 4171 D), manufactured by Gurley Precision Instruments of Troy, N.Y. The results are obtained in "Gurley Stiffness" values in units of milligrams.

## Claims

1. An absorbent article (1000, 3000) for being worn in a human body cleft, preferably between female labia,
between buttocks or gluteal clefts, or in the armpit,
said article consisting of compounds that are essentially fully biodegradable,
said article (1000, 3000) comprising
- a topsheet (1012, 3012) based on natural materials,
- an absorbent core (1015, 3041, 3051) comprising fibers and superabsorbent material,
- a backsheet (1018, 3018) based on natural or degradable materials,
wherein said topsheet, absorbent core, or backsheet comprise compounds selected from the group consisting of
- cellulose fibers or modified cellulose materials;
- starch, starch based or starch derived compounds;
- polylactic acid or polylactates;
- polyhydroxy-butyrate, -alkanoate, or -valerate;
- hydroxymethylfurfural;
- polycaprolactone;
- polyamide;
- polyurethane;
said article (1000, 3000) exhibiting
- an overall length (1001) of
more than 50 mm, preferably more than 70 mm,
but less than 150mm, preferably less than 120 mm, more preferably less than 110 mm,
- an overall width (1007) of more than 30 mm,
preferably more than 40 mm, but less than 150
mm, preferably less than 130 mm,
wherein said article (1000, 3000)
comprises
- a fold line (1004, 3004) along its length and adapted to be positioned parallel to a sagittal plane of a wearer, thereby defining the longitudinal direction of the article, and
- a first (1010, 3040) and a second (1020, 3050) side portion extending away from said fold line in the width direction of the article,
wherein each of said side portions
comprises
- a first surface formed by said topsheet (1012, 3012);
- a second surface opposite of said first surface, formed by said backsheet (1018, 3()18);
- and an absorbent core positioned between said first and said second surface;
said article being essentially free of adhesives, comprising thermofusible bonding only, wherein further
said article (1000, 3000) or its absorbent core (1015, 3041, 3051) satisfying at least one of the following conditions when tested according to the Folded Drip Off Capacity test as described herein:
- for the article
∘ a high pressure drip off capacity under 3.92 kPa (also expressed as 40 g/cm² load) of at least 2 ml;
∘ low pressure drip off capacity under 0.981 kPa (also expressed as 10 g/cm²) of at least 4 ml;
∘ high pressure edge wetting of no more than 0.1 g at a test fluid load of 80 % of high pressure drip off capacity under 3.92 kPa;
∘ low pressure edge wetting of no more than 0.1 g at a test fluid load of 80 % of low pressure drip off capacity under 0.981 kPa;
- for the absorbent core
∘ a high pressure core drip off capacity factor under 3.92 kPa (also expressed as 40 g/cm² load) of at least 2 ml / g of core material;
∘ a low pressure core drip off capacity factor under 0.981 kPa (also expressed as 10 g/cm² load) of at least 4 ml per gram of core material.

2. An absorbent article (1000, 3000) according to any of the preceding claims, wherein further
i) said topsheet (1012, 3012)
comprises one or more elements of the group consisting of
- fibers comprising cotton or viscose;
- apertured film materials, based on biodegradable materials;
- coating materials, based on biodegradable materials;
- binder materials, based on biodegradable materials;
ii) said absorbent core (1015, 3041, 3051)
comprises one or more elements of the group consisting of
- fibers being biodegradable,
- superabsorbent material being biodegradable,
- binder materials, being biodegradable materials;
iii) said backsheet (1018, 3018)
comprises one or more elements of the group consisting of
- fibers, preferably comprising cotton or viscose
- biodegradable film materials;
- biodegradable coating materials;
- biodegradable binder materials.

3. An absorbent article according to any of claims 1 to 2, further comprising one or more of the elements selected from the group consisting of
iv) a removal means (1110), preferably strings, stripes or bands;
v) an application aid, preferably a finger pouch;
vi) an attachment means (1120) for temporarily connecting a surface of the article to other surfaces, preferably of garments or skin of the wearer;
vii) an emollient / lotion applied to the first surface (1130);
viii) edge barrier webs (1180);
ix) a barrier compound applied to the edges, preferably a wax;
x) an absorbent core (1015, 3041, 3051) that is an oval shaped core comprising two sub-layers;
xi) being part of a series of articles, preferably wrapped articles, wound up in a roll, said roll preferably exhibiting the width of conventional toilet paper rolls, said series of articles being arranged in a single or multi lane arrangement, preferably a staggered multi-lane arrangement.

4. An absorbent article (1000, 3000) according to any of the preceding claims, comprising an absorbent core (1015, 3041, 3051) comprising
- a first piece of a first absorbent web material, and
- a second piece of a second absorbent web material,
each of said first and said second pieces exhibiting
a first and a second end edge
opposite along the longitudinal direction of the absorbent core,
connected by longitudinally extending side edges,
wherein said first and said second piece being positioned z-directionally in a partly overlapping arrangement, and longitudinally in an offset arrangement.

## Patentansprüche

1. Absorbierender Artikel (1000), 3000) zum Tragen in einem menschlichen Körperspalt, vorzugsweise zwischen weiblichen Schamlippen, zwischen Gesäßbacken oder in der Gesäßfalte oder in der Achselhöhle,
wobei der Artikel aus Verbindungen besteht, die im Wesentlichen vollständig biologisch abbaubar sind,
wobei der Artikel (1000, 3000) umfasst:
- eine auf natürlichen Materialien basierte Oberschicht (1012, 3012);
- einen absorbierenden Kern (1015, 3041, 3051) enthaltend Fasern und superabsorbierendes Material
- eine auf natürlichen Materialien basierte Unterschicht (1018. 3018)
wobei die Oberschicht, der absorbierende Kern oder die Unterschicht Komponenten aus der folgenden Gruppe enthalten:
- Zellulosefasern oder modifiziertes Zellulosematerial;
- Stärke, stärkebasierte Materialien oder Stärkederivate;
- Polymilchsäuren oder Polylactate;
- Polyhydroxy-butyrate, - alkanoate, oder -valerate;
- Hydroxymethylfurfural;
- Polycaprolactone;
- Polyamid;
- Polyurethan;
wobei der Artikel (1000, 3000) folgende Maße aufweist
- eine Gesamtlänge (1001) von mehr als 50 mm, vorzugsweise mehr als 70 mm, aber weniger als 150 mm, vorzugsweise weniger als 120 mm, noch bevorzugterweise weniger als 110 mm,
- eine Gesamtbreite (1007) von mehr als 30 mm, vorzugsweise mehr als 40 mm, aber weniger als 150 mm, vorzugsweise weniger als 130 mm,
wobei der Artikel (1000, 3000) ferner umfasst
- eine Faltlinie (1004, 3004), die angepasst ist, um parallel zu einer Sagittalebene eines Trägers positioniert zu werden, wodurch die Längsrichtung des Artikels definiert wird, und
- einen ersten (1010, 3040) und einen zweiten (1020, 3050) Seitenabschnitt, die sich von der Faltlinie in der Breitenrichtung des Artikels weg erstrecken,
wobei jeder der Seitenabschnitte umfasst:
- eine erste Oberfläche, die durch die Oberschicht (1012, 3012) gebildet wird;
- eine der ersten Oberfläche gegenüberliegende zweite Oberfläche, die durch die Unterschicht (1018, 3018) gebildet wird;
- und einen absorbierenden Kern, der zwischen der ersten und der zweiten Oberfläche positioniert ist;
wobei der Artikel im Wesentlichen leimfrei ist und nur thermische Schmelzverbindungen enthält, wobei der Artikel (1000, 3000) oder sein absorbierender Kern (1015, 3041, 3051) mindestens eine der folgenden Bedingungen erfüllt, wenn sie gemäß dem hierin beschriebenen gefalteten Abtropfkapazitätstest getestet wurden:
- für den Artikel:
o eine Hochdruck-Tropfkapazität unter 3,92 kPa (auch ausgedrückt als 40 g/cm2 Last) von mindestens 2 ml;
o eine Niederdruck-Tropfkapazität unter 0,981 kPa (auch ausgedrückt als 10 g/cm2) von mindestens 4 ml;
o eine Hochdruck-Randbenetzung von nicht mehr als 0,1 g bei einer Testfluidbeladung von 80% der Hochdruck-Tropfkapazität unter 3,92 kPa;
o eine Niederdruck-Randbenetzung von nicht mehr als 0,1 g bei einer Testfluidbeladung von 80% der Niederdruck-Tropfkapazität unter 0,981 kPa,
- für den absorbierenden Kern
o einen Hochdruck-Kernabtropfkapazitätsfaktor unter 3,92 kPa (auch ausgedrückt als 40 g/cm2 Last) von mindestens 2 ml/g Kernmaterial;
o einen Niederdruck-Kernabtropfkapazitätsfaktor unter 0,981 kPa (auch ausgedrückt als 10 g/cm2 Belastung) von mindestens 4 ml pro Gramm Kernmaterial.

2. Absorbierender Artikel (1000, 3000) nach einem der vorhergehenden Ansprüche, ferner umfassend
i) die Oberschicht (1012, 3012)
umfassend ein oder mehrere Elemente der Gruppe bestehend aus
- Fasern, die Baumwolle oder Viskose enthalten;
- gelochte Folienmaterialien auf der Basis von biologisch abbaubaren Materialien;
- Beschichtungsmaterialien auf der Basis von biologisch abbaubaren Materialien;
- Bindemittel auf der Basis von biologisch abbaubaren Materialien;
ii) einen absorbierenden Kern (1015, 3041, 3051)
umfassend ein oder mehrere Elemente der Gruppe bestehend aus
- biologisch abbaubare Fasern,
- biologisch abbaubares superabsorbierendes Material,
- biologisch abbaubare Bindemittel;
iii) eine Unterschicht (1018, 3018)
umfassend ein oder mehrere Elemente der Gruppe bestehend aus
- Fasern, vorzugsweise Baumwolle oder Viskose enthaltend;
- biologisch abbaubaren Folienmaterialien;
- biologisch abbaubaren Beschichtungsmaterialien;
- biologisch abbaubaren Bindemittel.

3. Absorptionsartikel nach einem der Ansprüche 1 bis 2, ferner umfassend eines oder mehrere der Elemente, ausgewählt aus der Gruppe bestehend aus
iv) Entfernungsmittel (1110), vorzugsweise Fäden, Streifen oder Bänder;
v) einer Applikationshilfe, vorzugsweise eine Fingertasche;
vi) ein Befestigungsmittel (1120) zum temporären Verbinden einer Oberfläche des Artikels mit anderen Oberflächen, vorzugsweise von Kleidungsstücken oder der Haut des Trägers;
vii) ein(e) Emolliens / Lotion, zum Aufbringen auf die erste Oberfläche (1130);
viii) ein Randsperrschichtmaterial
ix) eine Sperrschicht (1180), die auf die Ränder aufgebracht ist, vorzugsweise ein Wachs;
x) einen absorbierenden Kern (1015, 3041, 3051), der ein ovaler geformter Kern ist, der zwei Unterschichten umfasst;
xi) Teil einer Reihe von Artikeln, vorzugsweise umhüllten Artikeln, die in einer Rolle aufgewickelt sind, wobei die Rolle vorzugsweise die Breite herkömmlicher Toilettenpapierrollen aufweist, wobei die Reihe von Artikeln in einer Ein- oder Mehrspuranordnung angeordnet ist, vorzugsweise eine versetzte mehrspurige Anordnung.

4. Absorbierender Artikel (1000, 3000) nach einem der vorhergehenden Ansprüche, umfassend einen saugfähigen Kern (1015, 3041, 3051) mit
- einem ersten Stück eines ersten saugfähigen Bahnmaterials, und
- einem zweiten Stück eines zweiten saugfähigen Bahnmaterials,
wobei jedes der ersten und zweiten Stücke Folgendes aufweist
eine erste und eine zweite Seitenkante,
die sich entlang der Längsrichtung des absorbierenden Kerns gegenüberliegen,
die durch sich in Längsrichtung erstreckende Seitenkanten verbunden sind,
wobei das erste und das zweite Stück in z-Richtung in einer teilweise überlappenden Anordnung und in Längsrichtung in einer versetzten Anordnung positioniert sind.

## Revendications

1. Article absorbant (1000, 3000) pour être porté dans une fente de corps humain, de préférence entre des lèvres de la vulve femelles, entre des fesses ou dans le pli interfessier, ou dans l'aisselle, ledit article étant constitué de composés qui sont essentiellement entièrement biodégradable,
ledit article (1000, 3000) comprenant
- une feuille de dessus (1012, 3012) à base de matériaux naturels,
- un noyau absorbant (1015, 3041, 3051) comprenant des fibres et de matériau superabsorbant,
- une feuille de fond (1018, 3018) à base de matériaux naturels,
dans lequel la feuille de dessus, le noyau absorbant ou la feuille de fond comprend des constituants sélectionnées du groupe constitué par
- du fibres cellulosique ou matières de la cellulose modifiée ;
- d'amidon, matériau à basé d'amidon ou de dérivés d'amidon ;
- d'acide polylactique ou des polylactates ;
- du Polyhydroxy-butyrate, - alcanoate, ou -valerate ;
- du hydroxymethylfurfural;
- du polycaprolactone;
- du polyamide;
- du polyuréthane;
l'article (1000, 3000) présentant
- une longueur totale (1001) de plus de 50 mm, de préférence plus de 70 mm, mais inférieure à 150 mm, de préférence inférieure à 120 mm, plus préférablement inférieure à 110 mm,
- une largeur totale (1007) de plus de 30 mm, de préférence plus de 40 mm, mais inférieure à 150 mm, de préférence inférieure à 130 mm,
dans lequel ledit article (1000, 3000) comprend
- une ligne de pliage (1004, 3004) adaptée pour être positionnée parallèlement à un plan sagittal d'un porteur, définissant ainsi la direction longitudinale de l'article, et
- une première (1010, 3040) et une seconde (1020, 3050) partie latérale s'étendant à l'opposé de ladite ligne de pliage dans le sens de la largeur de l'article,
dans lequel chacune desdites parties latérales comprend
- une première surface étant formée par ladite feuille de dessus (1012, 3012)
- une seconde surface opposée à ladite première surface, formée par ladite une feuille de fond (1018, 3018);
- et un noyau absorbant positionné entre ladite première et ladite deuxième surface;
dans lequel ledit article est essentiellement exempt d'adhésifs, comprenant de préférence uniquement des liaisons thermofusibles,
ledit article (1000. 3000) ou son noyau absorbant (1015, 3041, 3051) satisfaisant au moins l'une des conditions suivantes lorsqu'il est testé selon le test plié de la capacité de dégouttement tel que décrit ici :
- pour l'article :
o une capacité de dégouttement à haute pression sous 3,92 kPa (également exprimée en tant que charge de 40 g/cm2) d'au moins 2 ml;
o une capacité de dégouttement à basse pression sous 0,981 kPa (également exprimée par 10 g/cm2) d'au moins 4 ml;
o un mouillage de bordure à haute pression inférieur ou égal à 0,1 g à une charge de fluide de test de 80% de capacité de dégouttement à haute pression sous 3,92 kPa;
o un mouillage de bordure à basse pression inférieur ou égal à 0,1 g à une charge de fluide d'essai de 80% de capacité d'arrêt à basse pression sous 0,981 kPa,
- pour le noyau absorbant
o un coefficient de capacité de dégouttement de noyau à haute pression sous 3,92 kPa (également exprimé en tant que charge de 40 g/cm2) d'au moins 2 ml/g de matériau de noyau;
o un coefficient de capacité de dégouttement de noyau à basse pression inférieur à 0,981 kPa (également exprimé en tant que charge de 10 g/cm2) d'au moins 4 ml par gramme de matériau de noyau.

2. Article absorbant (1000. 3000) selon l'une quelconque des revendications précédentes, comprenant en outre
i) ladite feuille dessus (1012, 3012)
comprenant un ou plusieurs éléments du groupe constitué par
- des fibres, à la base du coton ou viscose ;
- du matériau de film avec des apertures, à base du matériau biodégradable ;
- du matériau de revêtement, à base du matériau biodégradables ;
- du matériau liant, à base du matériau biodégradable ;
ii) ledit noyau absorbant (1015, 3041, 3051) comprenant un ou plusieurs éléments du groupe constitué par
- des fibres biodégradables,
- des matériaux superabsorbant biodégradable,
- des matériaux liants biodégradables ;
iii) une feuille de fond (1018, 3018)
comprenant un ou plusieurs éléments du groupe constitué par
- des fibres, de préférence comprenant coton ou viscose ;
- du matériau de film biodégradable ;
- des matériaux de revêtement biodégradables;
- des matériaux liants biodégradables.

3. Article absorbant selon la revendication 1 à 2, comprenant en outre un ou plusieurs des éléments choisis dans le groupe constitué par
iv) un moyen de retrait (1110), de préférence des cordes, des bandes ou des rubans ;
v) des auxiliaires d'application, de préférence une poche à doigts;
vi) un moyen de fixation (1120) pour relier temporairement une surface de l'article à d'autres surfaces, de préférence de vêtements ou de peau du porteur;
vii) un émollient/lotion appliqué sur la première surface (1130);
viii) des bandes barrières de bord;
ix) un couche barrière appliquée sur les bords, de préférence une cire;
x) un noyau absorbant (1015, 3041, 3051) qui est un noyau de forme ovale comprenant deux sous-couches;
xi) faisant partie d'une série d'articles, de préférence des articles enveloppés, enroulés en un rouleau, ledit rouleau présentant de préférence la largeur des rouleaux de papier hygiénique classiques, ladite série d'articles étant disposée dans un arrangement à une ou plusieurs voies, de préférence un arrangement à voies multiples en quinconce.

4. Article absorbant (1000, 3000) selon l'une quelconque des revendications précédentes, comprenant un noyau absorbant (1015, 3041, 3051) comprenant
- une première pièce d'un premier matériau absorbant, et
- une deuxième pièce d'un deuxième matériau absorbant,
chacune de ces première et deuxième pièces présentant
un premier et un second bord d'extrémité
opposés le long de la direction longitudinale du noyau absorbant,
reliées par des bords latéraux s'étendant longitudinalement,
la première et la seconde pièce étant positionnées dans le sens z selon une disposition partiellement chevauchante et dans le sens longitudinal selon une disposition décalée.
